(19) Europäisches Patentamt
European Patent Office
Office européen des brevets

(11) EP 2 206 491 B1

(12) FASCICULE DE BREVET EUROPEEN

(45) Date de publication et mention de la délivrance du brevet:
**17.08.2016 Bulletin 2016/33**

(51) Int Cl.:
***A61K 8/60*** *(2006.01)* ***A61Q 19/08*** *(2006.01)*

(21) Numéro de dépôt: **09181017.6**

(22) Date de dépôt: **30.12.2009**

(54) **Association de monosaccharides avec des dérivés C-glycosides et son utilisation en cosmétique**

Assoziation von Monosacchariden und C-Glycosidderivaten, und ihre Verwendung in der Kosmetik

Association of monosaccharides and C-glycoside derivatives and its use in cosmetics

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **30.12.2008 FR 0859150**
**15.01.2009 US 144754 P**

(43) Date de publication de la demande:
**14.07.2010 Bulletin 2010/28**

(73) Titulaire: **L'Oréal**
**75008 Paris (FR)**

(72) Inventeurs:
- **Laboureau, Julien**
**92130, Issy Les Moulineaux (FR)**
- **Simonnet, Jean-Thierry**
**94230, Cachan (FR)**
- **Portes, Pascal**
**94130, Nogent sur Marne (FR)**

(74) Mandataire: **Tezier Herman, Béatrice et al**
**Cabinet Becker & Associés**
**25, rue Louis le Grand**
**75002 Paris (FR)**

(56) Documents cités:
**EP-A- 1 589 010**
**WO-A-02/051828**
**WO-A-2008/148966**
**WO-A-2009/034559**
**FR-A- 2 609 397**
**FR-A- 2 756 735**
**FR-A- 2 768 623**
**FR-A- 2 773 324**
**FR-A- 2 876 283**
**FR-A1- 2 853 539**
**FR-A1- 2 900 574**
**FR-A1- 2 912 313**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 206 491 B1

## Description

La présente invention concerne une composition, notamment cosmétique et/ou dermatologique, comprenant dans un milieu physiologiquement acceptable, l'association d'au moins un monosaccharide choisi parmi le mannose, le rhamnose et leur mélange, et d'au moins un composé additionnel choisi parmi les C-glycosides, leurs dérivés et leurs mélanges. La présente invention concerne également l'utilisation d'une telle composition, ainsi qu'un dispositif la contenant.

La peau humaine est constituée principalement de deux couches principales qui sont le derme et l'épiderme qui recouvre le derme de manière superficielle. Le derme fournit à l'épiderme un support solide. C'est également son élément nourricier. Il est principalement constitué de fibroblastes et d'une matrice extracellulaire composée majoritairement de collagène, d'élastine et d'une substance, dite substance fondamentale. Ces composants sont synthétisés par les fibroblastes.

La cohésion entre l'épiderme et le derme est assurée par la jonction dermo-épidermique. C'est une région complexe d'une épaisseur de 100 nm environ qui comprend le pole basal des kératinocytes basaux, la membrane épidermique et la zone sous-basale du derme superficiel. D'un point de vue structurel, des hémidesmosomes, dans lesquels s'insèrent des filaments de kératine (complexe hémidesmosome-tonofilaments), sont répartis sur la membrane plasmique des kératinocytes basaux. Au regard de ces complexes hémidesmosome-tonofilaments, on trouve des filaments d'ancrage qui traversent la membrane basale épidermique. Les filaments d'ancrage sont reliés à la laminine V côté épidermique. Il a été montré que ces fibrilles d'ancrage, parfaitement visualisables en microscopie électronique, sont composées de collagène de type VII (ci-après collagène VII). Le collagène VII est synthétisé par les kératinocytes et les fibroblastes mais de façon plus importante par les kératinocytes (Aumailley M, Rousselle P. laminins of the dermoepidermal junction. Matrix Biology. 1999, 18: 19-28; Nievers M, Schaapveld R, Sonnenberg A. Biology and function of hemidesmosomes. Matrix Biology, 1999, 18 : 5-17).

Les collagènes sont les protéines majoritaires des matrices extracellulaires de la peau. A ce jour, 20 types de collagènes sont identifiés et notés de I à XX. Les collagènes ne sont pas tous synthétisés par les mêmes types cellulaires, la régulation de leur expression diffère d'un collagène à un autre, et toutes les molécules de collagène sont des variantes d'un précurseur commun qui est la chaîne alpha du procollagène.

La jonction dermo-épidermique est une structure qui conditionne l'état de surface de la peau. Ainsi, une jonction dermo-épidermique présentant des structures d'ancrage intègres est maintenue plissée, permettant ainsi d'augmenter la surface de la zone de contact entre le derme et l'épiderme, de favoriser les échanges de facteurs diffusibles notamment entre ces deux tissus, de renforcer leur cohésion et d'améliorer l'apparence de l'épiderme. Dans des cas où les structures d'ancrage sont altérées, en particulier du fait d'une déficience de la synthèse du collagène IV, du collagène VII, de laminine V et/ou du fait du vieillissement, ceci provoque un aplanissement de la jonction dermo-épidermique. Les échanges sont amoindris, les deux tissus sont moins solidaires, l'épiderme se plisse et la peau étant moins ferme et moins tendue, les rides apparaissent et la fragilité de la peau aux agressions mécaniques est accrue.

Avec le vieillissement, le collagène s'amincit et se désorganise, le renouvellement des cellules de la peau est diminué, des rides apparaissent à la surface de la peau, la peau est moins ferme, plus terne. Le vieillissement cutané est conditionné par des caractéristiques génétiques. Par ailleurs, certains facteurs d'environnement comme le tabagisme et surtout l'exposition au rayonnement du soleil l'accélèrent. La peau a ainsi un aspect beaucoup plus âgé sur les zones exposées au soleil comme le dos des mains ou le visage. Ainsi, ces autres facteurs ont également un impact négatif sur le collagène naturel de la peau.

En conséquence, compte tenu du rôle important du collagène au niveau de l'intégrité de la peau et de sa résistance aux agressions externes de type mécaniques, la stimulation de synthèse de ces collagènes, et en particulier du procollagène I, collagène IV, du collagène VII, et de la laminine V, apparaît comme un moyen efficace pour pallier les signes du vieillissement cutané.

Pour pallier les inconvénients précités, pour améliorer l'apparence de la peau, pour améliorer ses propriétés mécaniques et éviter les pathologies associées à la carence ou à la déficience cellulaire, du renouvellement cellulaire, ou de certains composés du derme ou de la jonction dermo-épidermique, il semble important de développer des produits qui visent à renforcer ou maintenir le rôle de support et d'élément nourricier que joue le derme, la cohésion entre les différentes couches de la peau, et plus particulièrement la cohésion entre le derme et l'épiderme, en augmentant la prolifération des kératinocytes, en stimulant la prolifération et le métabolisme des fibroblastes et en stimulant la synthèse des collagènes, en particulier le procollagène I, le collagène IV, le collagène VII, et la laminine V.

L'épiderme qui recouvre le derme, et est en contact direct avec l'environnement extérieur, a comme rôle principal de protéger l'organisme de la déshydratation et des agressions extérieures. L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau.

Le derme est principalement constitué de fibroblastes et d'une matrice extracellulaire. On y trouve aussi des

leucocytes, des mastocytes ou encore des macrophages tissulaires. Il est également constitué de vaisseaux sanguins et de fibres nerveuses.

La matrice extracellulaire du derme, comme celle de tous les tissus conjonctifs de l'organisme, est composée de protéines appartenant à plusieurs grandes familles les collagènes, les glycoprotéines matricielles autres que les collagènes (fibronectine, laminine), l'élastine et les protéoglycannes. On trouve également dans la matrice extracellulaire du derme, comme celle de tous les tissus conjonctifs de l'organisme, des glycosaminoglycannes sous forme libre (c'est à dire non liés à une protéine).

Il est maintenant bien établi que des interactions spécifiques existent entre ces différentes classes de protéines pour donner naissance à un tissu fonctionnel.

Les protéoglycannes sont des macromolécules complexes constituées d'un tronc protéique central ramifié, ou réseau de protéines, auquel sont attachés de très nombreuses chaînes latérales polyosidiques appelées glycosaminoglycannes.

Dans la suite de la présente demande, on désignera les protéoglycannes par l'abréviation PGs et les glycosaminoglycannes par l'abréviation GAGs.

Les GAGs ont longtemps été désignés sous le terme de mucopolysaccharides acides en raison de leur forte capacité de rétention d'eau, de leur nature glucidique et de leur caractère acide provenant de leurs multiples charges négatives.

Ainsi, la polarité des GAGs les fait implicitement participer à certaines fonctions biologiques comme l'hydratation des tissus, la fixation des cations ou le rôle de barrière de filtration ionique.

Les PGs et les GAGs sont synthétisés par différentes cellules au niveau du derme et de l'épiderme : fibroblastes, kératinocytes et mélanocytes.

Les fibroblastes synthétisent majoritairement des collagènes, des glycoprotéines matricielles autres que les collagènes (fibronectine, laminine), des protéoglycannes et de l'élastine. Les kératinocytes synthétisent majoritairement des GAGs sulfatés et de l'acide hyaluronique alors que les mélanocytes ne produisent apparemment pas d'acide hyaluronique.

Lorsqu'ils sont incorporés dans un PG, les GAGs sont des chaînes linéaires composées de la répétition d'un diholoside de base contenant toujours une hexosamine (glucosamine ou galactosamine) et un autre ose (acide glucuronique, acide iduronique ou galactose). La glucosamine est soit N-sulfatée, soit N-acétylée. En revanche, la galactosamine est toujours N-acétylée. En outre, il peut y avoir des sulfates O-liés sur l'hexosamine, l'acide uronique et le galactose.

Le fort caractère anionique des GAGs s'explique par la présence de groupes carboxylates au sein des acides hexuroniques (acide glucuronique et acide iduronique) et de groupes sulfates O- et N-liés.

Les principaux GAGs sont l'acide hyaluronique ou hyaluronanne (HA), l'héparane sulfate (HS), l'héparine (HP), la chondroïtine, la chondroftine sulfate (CS), la chondroïtine 4-sulfate ou chondroïtine sulfate A (CSA), la chondroïtine 6-sulfate ou chondroïtine sulfate C (CSC), le dermatane sulfate ou chondroïtine sulfate B (CSB) et le kératane sulfate (KS) qui diffère des autres glycosaminoglycannes par la présence de galactose à la place de l'acide uronique.

Lorsqu'ils sont associés à une protéine sous forme de PG, les GAGs sont liés par des structures d'ancrage aux différentes chaînes polypeptidiques, nommées protéine core ou protéine porteuse, et forment ainsi des molécules de PGs.

Les GAGs peuvent également exister dans la matrice extracellulaire sous forme libre c'est à dire non liée à une protéine matricielle : c'est notamment le cas de l'acide hyaluronique.

Lors de la synthèse des PGs, les GAGs sont polymérisés à partir de ces structures d'ancrage.

La synthèse des GAGs nécessite l'action coordonnée et concertée d'enzymes très spécifiques (transférases, épimérases, sulfotransférases) adjacentes dans la membrane du réticulum endoplasmique et de l'appareil de Golgi. Puis une multitude de réactions biochimiques (N-désacétylation, N- et O- sulfatations, épimérisation) modifient les deux oses constitutifs du motif de base et cela de manière hétérogène le long de la chaîne. D'une chaîne d'héparane sulfate à l'autre par exemple, le rapport acide glucuronique/acide iduronique, la nature, le nombre et la position des O-sulfatations, ainsi que le rapport N-sulfate/O-sulfate peuvent varier, ce qui, potentiellement offre une immense diversité structurale.

De façon générale, les rôles biologiques des PGs sont très diversifiés, allant d'une fonction passive de support mécanique (par exemple serglycines) ou d'un rôle de barrière ionique de filtration moléculaire (par exemple perlecanne et bamacanne de la membrane basale glomérulaire), à des effets plus spécifiques dans l'adhésion, l'étalement, la prolifération, la différenciation cellulaire ou la morphogenèse, ou, à des effets très spécifiques d'interactions PG-protéine, tels que la fonction de récepteur du bétaglycanne ou l'interaction de la décorne avec le collagène.

Un des rôles du tissu conjonctif dermique est de protéger l'organisme contre les agressions externes en formant en même temps une interface informative.

Pour ce faire, le derme possède une forte résistance mécanique en gardant, toutefois, une grande souplesse.

Sa résistance est assurée par le réseau dense des fibres de collagène, mais ce sont les PGs et l'acide hyaluronique, en assurant l'hydratation, la distribution et la souplesse des fibres qui font la différence entre la peau et, par

exemple, le cuir.

Les PGs constituent 0,5 à 2 % du poids sec du derme, le collagène en représentant à lui seul jusqu'à 80 %.

La concentration et la distribution dans la peau humaine des GAGs et des PGs varient avec l'âge.

L'acide hyaluronique ou hyaluronanne (HA) est le principal GAG du derme, ce dernier renfermant la moitié d'HA de l'organisme.

La synthèse d'HA est effectuée notamment par les fibroblastes, à proximité de la face interne de la membrane plasmique. Elle s'effectue de façon continue. Ce polysaccharide gigantesque (plusieurs millions de daltons) possède une viscosité intrinsèque très élevée, assurant l'hydratation et l'assemblage des différents éléments du tissu conjonctif par formation de complexes supramoléculaires.

Le dermatanne sulfate (DS), initialement isolé du derme, est aussi très abondant dans la peau. Il constitue 40 à 50 % des GAGs dermiques.

Parallèlement aux mécanismes contribuant à l'élaboration de ces matrices extracellulaires spécialisées, il existe des processus de remodelage continuel dont la régulation dépend de la balance entre synthèse et dégradation des éléments protéiques de la matrice.

Plusieurs familles de protéases matricielles sont maintenant décrites ainsi que les facteurs intervenant dans leur activation-inactivation.

Au cours du vieillissement chronologique et/ou actinique, le derme et l'épiderme subissent de nombreuses modifications et dégradations qui se traduisent, avec l'âge, par une flaccidité et une perte de souplesse cutanée.

Parmi les éléments dégradés (notamment collagène et élastine), les PGs et les GAGs sont également altérés. En effet, au cours du vieillissement, la production par les fibroblastes et les kératinocytes des PGs et des GAGs est altérée. Il en résulte une désorganisation importante : le dépôt des GAGs sur le squelette protéique formant le PG est anormal, ce qui a pour conséquence une moins grande avidité pour l'eau de ces PGs et donc une diminution de l'hydratation et de la tonicité des tissus.

Restaurer une production normale de PGs et de GAGs par les fibroblastes et les kératinocytes contribue, en partie, à compenser la perte en hydratation cutanée.

La dégradation de ces matrices contribue donc au phénomène d'assèchement et de perte de souplesse de la peau.

On comprend donc l'importance de pouvoir disposer de produits dont les effets visent à maintenir le taux de PGs et de GAGs dans la peau et lui maintenir ainsi entre autre une bonne hydratation et une bonne souplesse en luttant efficacement contre les signes du vieillissement.

A cet égard la demanderesse a découvert, de manière surprenante et inattendue, que l'association d'au moins un monosaccharide choisi parmi le mannose, le rhamnose et leur mélange, et d'au moins un composé additionnel choisi parmi les C-glycosides, leurs dérivés et leurs mélanges, entraîne une complémentarité d'action à la fois sur l'augmentation de l'épaisseur de l'épiderme, sur le renforcement de la structure de la jonction dermo-épidermique, en particulier en augmentant la synthèse des collagène IV et VII et/ou des laminines telles que la laminine V, et/ou sur la stimulation de la synthèse du procollagène I et sur l'activation de la prolifération des kératinocytes et/ou des fibroblastes. La complémentarité d'action de l'association selon l'invention stimule la synthèse des GAGs contenant un résidu D-glucosamine et/ou N-acétyl D-glucosamine, avantageusement de l'acide hyaluronique, et/ou des PGs, avantageusement des protéoglycannes contenant de l'acide hyaluronique, par les fibroblastes et/ou les kératinocytes, dont le métabolisme et la prolifération sont également stimulés. Les effets obtenus avec l'association définie ci-dessus, engendrent donc une régénération de la peau en agissant à la fois sur l'épiderme, la jonction dermo-épidermique et sur le derme, démontrant ainsi une activité anti-âge globale. En particulier, la combinaison de monosaccharide selon l'invention avec un C-glycoside a permis d'obtenir un effet à la fois sur les cellules et la matrice les environnant. Ainsi, cette combinaison a permis à la fois la stimulation et/ou la prolifération des fibroblastes, et une production accrue de glycosaminoglycanes qui permet d'améliorer la matrice extracellulaire.

La demanderesse a en effet démontré l'activation de la prolifération des kératinocytes et des fibroblastes et la stimulation de la synthèse du procollagène I par le mannose ou le rhamnose. L'utilisation de compositions les contenant permet ainsi de contrer les signes du vieillissement cutané, et en particulier l'atrophie épidermique et/ou dermique liée au vieillissement.

L'utilisation de ces monosaccharides était restée jusqu'alors inconnue pour les effets biologiques directs exposés plus haut. La demande WO 2007/128939 mentionne toutefois une activité anti-âge obtenue par un effet biomécanique d'un agent tenseur en association avec des composés saccharidiques, qui permettent d'augmenter l'expression des mécanorécepteurs de cellules de la peau. Cette augmentation de l'expression des mécanorécepteurs est décrite comme augmentant la sensibilisation des cellules de la peau à répondre aux effets des tenseurs.

La demande WO 2005/063194 décrit une base galénique à très haute tolérance comprenant notamment du mannose ou du rhamnose. Il est spécifié qu'une telle base galénique ne peut fonctionner qu'en association avec un actif dont elle est seulement le véhicule. Les bases galéniques dermiques et/ou cosmétiques divulguées reposent essentiellement sur la présence des deux polyols que sont le mannitol et le xylitol.

D'autre part, l'influence des C-Glycosides sur la synthèse des GAGs et des PGs est décrite dans la demande WO 2002/051828. Ce document décrit également l'influence des C-Glycosides sur l'amélioration de la structure tridimensionnelle de la jonction dermo-épidermique qui assurent à ce niveau le renforcement de la liaison entre la laminine VI et le nidogène, avec notamment une action au niveau du collagène IV.

L'influence de certains dérivés C-xylosidés sur la synthèse des GAGs et sur la régulation de l'expression de certaines molécules de la jonction dermo-épidermique est connu de la littérature (Eur. J. Dermatol., 2008, 18(1), 36-40 et Eur. J. Dermatol., 2008, 18(2), 297-302).

La présente invention concerne donc une composition, notamment cosmétique et/ou dermatologique, comprenant l'association d'au moins un monosaccharide choisi parmi le mannose, le rhamnose et leur mélange avec au moins un composé additionnel choisi parmi les C-glycosides, leurs dérivés et leurs mélanges.

Le mannose est un hexose épimère en C2 du glucose. Le rhamnose (ou 6 deoxy mannose) constitue formellement le produit de désoxygénation du mannose en C6. Les monosaccharides selon l'invention sont sous la forme D ou L du mannose et/ou du rhamnose ou leur mélange, chaque forme pouvant elle-même être l'anomère alpha et/ou béta. Les formes préférées selon l'invention sont le D-mannose et/ou le L-rhamnose.

Le D-mannose est présent dans les végétaux en particulier certains fruits dont les airelles (canneberges), ou le bois dur (hêtre, bouleau). Le rhamnose est trouvé dans la nature sous forme L. Le D-mannose, ainsi que le L-rhamnose, sont commercialisés, par exemple, par la société Danisco Sweeteners®. Dans la présente invention, le monosaccharide est plus spécifiquement sous forme de monomère.

De manière préférée, la composition selon l'invention comprend un C-glycoside répondant à la formule (I) suivante :

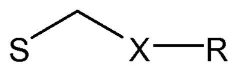

(I)

dans laquelle,

- R représente :

  - un radical alkyle linéaire, saturé en C1 à C20, en particulier en C1 à C10, ou insaturé en C2 à C20, en particulier en C2 à C10, ou un radical alkyle ramifié ou cyclique, saturé ou insaturé, en C3 à C20, en particulier en C3 à C10 ;
  - un radical hydrofluoro- ou perfluoro-alkyle, linéaire saturé en C1 à C20, en particulier en C1 à C10, ou insaturé en C2 à C20, en particulier en C2 à C10, ou ramifié ou cyclique, saturé ou insaturé, en C3 à C20, en particulier en C3 à C10 ; la chaîne hydrocarbonée constituant lesdits radicaux pouvant, le cas échéant, être interrompue par 1, 2, 3 ou plus d'hétéroatomes choisis parmi :
  - un oxygène,
  - un soufre,
  - un azote, et
  - un silicium,

  et pouvant être éventuellement substituée par au moins un radical choisi parmi :

  - -OR4,
  - -SR4,
  - -NR4R5,
  - -COOR4,
  - -CONHR4,
  - -CN,
  - un atome d'halogène,
  - un radical hydrofluoro- ou perfluoro-alkyle, en C1 à C6, et/ou
  - un radical cycloalkyle en C3 à C8,

  avec R4 et R5 pouvant représenter, indépendamment l'un de l'autre, un atome d'hydrogène, ou un radical alkyle, perfluoroalkyle ou hydrofluoroalkyle linéaire, saturé en C1 à C30, notamment en C1 à C12, ou insaturé en C2 à C30, notamment en C2 à C12, ou ramifié ou cyclique, saturé ou insaturé, en C3 à C30, notamment en C3 à C12 ; ou un radical aryle en C6 à C10,

- X représente un radical choisi parmi les groupements :

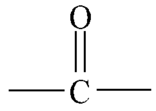

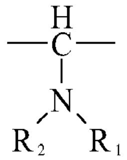

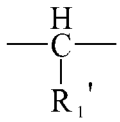

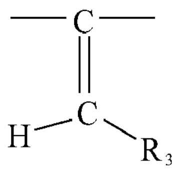

avec R1, R2 et R3 représentant, indépendamment l'un de l'autre, un atome d'hydrogène, ou un radical R, avec R tel que défini précédemment, et R'1 représente un atome d'hydrogène, un groupe -OH ou un radical R tel que défini précédemment, R1 pouvant désigner également un radical aryle en C6 à C10 ;

- S représente un monosaccharide ou un polysaccharide comportant jusqu'à 20 unités sucre, en particulier jusqu'à 6 unités sucre, sous forme pyranose et/ou furanose et de série L et/ou D, ledit mono- ou poly-saccharide pouvant être substitué par un groupement hydroxyle obligatoirement libre, et éventuellement une ou plusieurs fonction(s) amine(s) éventuellement protégée(s), et

- la liaison S-CH2-X représente une liaison de nature C-anomérique, qui peut être [alpha] ou [beta],

ainsi que leurs sels cosmétiquement acceptables, leurs solvates tels que les hydrates et leurs isomères.

Dans le cadre de la présente invention, par "halogène", on entend le chlore, le fluor, le brome ou l'iode.

Le terme "aryle" désigne un cycle aromatique tel que phényle, éventuellement substitué par un ou plusieurs radicaux alkyles en C1-C4.

Le terme "cycloalkyle en C3 à C8 " désigne un cycle aliphatique ayant de 3 à 8 atomes de carbone, incluant par exemple le cyclopropyle, le cyclopentyle et le cyclohexyle.

Parmi les groupes alkyles convenant à la mise en oeuvre de l'invention, on peut notamment citer les groupes méthyle, éthyle, isopropyle, n-propyle, n-butyle, t-butyle, isobutyle, sec-butyle, pentyle, n-hexyle, cyclopropyle, cyclopentyle, cyclohexyle, et allyle.

Selon un mode de réalisation de l'invention, on peut utiliser un dérivé C-glycoside répondant à la formule (I) pour lequel S peut représenter un monosaccharide ou un polysaccharide contenant jusqu'à 6 unités sucre, sous forme pyranose et/ou furanose et de série L et/ou D, ledit mono- ou polysaccharide présentant au moins une fonction hydroxyle obligatoirement libre et/ou éventuellement une ou plusieurs fonctions aminées obligatoirement protégées, X et R conservant par ailleurs l'ensemble des définitions précédemment données.

Avantageusement, un monosaccharide de l'invention peut être choisi parmi le D-glucose, le D-galactose, le D-mannose, le D-xylose, le D-lyxose, le L-fucose, le L-arabinose, le L-rhamnose, l'acide D-glucuronique, l'acide D-galacturonique, l'acide D-iduronique, la N-acétyl-D-glucosamine, la N-acétyl-D-galactosamine et désigne avantageusement le D-glucose, le D-xylose, la N-acétyl-D-glucosamine ou le L-fucose, et en particulier le D-xylose.

Plus particulièrement, un polysaccharide de l'invention contenant jusqu'à 6 unités sucres peut être choisi parmi le D-maltose, le D-lactose, le D-cellobiose, le D-maltotriose, un disaccharide associant un acide uronique choisi parmi l'acide D-iduronique ou l'acide D-glucuronique avec une hexosamine choisie parmi la D-galactosamine, la D-glucosamine, la N-acétyl-D-galactosamine, la N-acétyl-D-glucosamine, un oligosaccharide contenant au moins un xylose qui peut être avantageusement choisi parmi le xylobiose, le méthyl-[beta]-xylobioside, le xylotriose, le xylotétraose, le xylopentaose et le xylohexaose et notamment le xylobiose qui est composé de deux molécules de xylose liées par une liaison 1-4.

Plus particulièrement, S peut représenter un monosaccharide choisi parmi le D-glucose, le D-xylose, le L-fucose, le D-galactose, le D-maltose et notamment le D-xylose. Selon un autre mode de réalisation de l'invention, on peut utiliser des dérivés C-glycosides répondant à la formule (I) pour lesquels X représente un groupement choisi parmi -CO-, -CH(OH)-, -CH(NR1R2)-, -CH(R)-, en particulier -CO-, - CH(OH)-, -CH(NH2)-, -CH(NHCH2CH2CH2OH)-, -CH(NHPh)-, -CH(CH3)-, et plus particulièrement un groupement -CO-, -CH(OH)-, -CH(NH2)-, et préférentiellement un groupement -CH(OH)-, S et R conservant par ailleurs l'ensemble des définitions précédemment données.

Selon un autre mode de réalisation de l'invention, on peut utiliser un dérivé C-glycoside répondant à la formule (I) pour lesquels R représente un radical alkyle linéaire, saturé en C1 à C20, en particulier en C1 à C10, ou insaturé en C2 à C20, en particulier en C2 à C10, ou un radical alkyle ramifié ou cyclique, saturé ou insaturé, en C3 à C20 ; en particulier en C3 à C10, et éventuellement substitué comme décrit précédemment, S et X conservant par ailleurs l'ensemble des définitions précédemment données. De préférence, R désigne un radical linéaire en C1-C4, notamment C1-C3, éventuellement substitué par -OH, -COOH ou - COOR"2, R"2 étant un radical alkyle saturé en C1-C4, notamment éthyle.

Préférentiellement R désigne un radical alkyle linéaire non substitué en C1-C4, notamment C1-C2, en particulier éthyle.

Parmi les dérivés C-glycosides de formule (I), on utilise de préférence ceux pour lesquels :

- R représente un radical alkyle linéaire, saturé en C1 à C20, en particulier en C1 à C10, ou insaturé en C2 à C20, en particulier en C2 à C10, ou un radical alkyle ramifié ou cyclique, saturé ou insaturé, en C3 à C20 ; en particulier en C3 à C10, et éventuellement substitué comme décrit précédemment ;
- S représente un monosaccharide comme décrit précédemment ;
- X représente -CO-, -CH(OH)-, -CH(NR1 R2)-, -CH(R)- comme décrit précédemment.

De préférence, on utilise un dérivé C-glycoside de formule (I) pour lesquels

- R désigne un radical linéaire en C1-C4, notamment C1-C3, éventuellement substitué par -OH, -COOH ou -COOR"2, R"2 étant un radical alkyle saturé en C1-C4, notamment éthyle ;
- S représente un monosaccharide comme décrit précédemment ;
- X représente un groupement choisi parmi -CO-, -CH(OH)-, -CH(NH2)-, -CH(NHCH2CH2CH2OH)-, -CH(NHPh)-, -CH(CH3)-, et plus particulièrement un groupement -CO-, -CH(OH)-, -CH(NH2)-, et préférentiellement un groupement -CH(OH)-

Préférentiellement, on utilise un dérivé C-glycoside de formule (I) pour lequel :

- R désigne un radical alkyle linéaire non substitué en C1-C4, notamment C1-C2, en particulier éthyle ;
- S représente un monosaccharide comme décrit précédemment ; notamment le D-glucose, le D-xylose, la N-acétyl-D-glucosamine ou le L-fucose, et en particulier le D-xylose ;
- X représente un groupement choisi parmi -CO-, -CH(OH)-, -CH(NH2)-, et préférentiellement un groupement -CH(OH)-.

Les sels acceptables pour l'usage non thérapeutique des composés décrits dans la présente invention comprennent des sels non toxiques conventionnels desdits composés tels que ceux formés à partir d'acides organiques ou inorganiques. A titre d'exemples, on peut citer les sels d'acides minéraux, tels que l'acide sulfurique, l'acide chlorhydrique, l'acide bromhydrique, l'acide iodhydrique, l'acide phosphorique, l'acide borique. On peut également citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyles. On peut notamment citer l'acide propionique, l'acide acétique, l'acide téréphtalique, l'acide citrique et l'acide tartrique.

Lorsque le composé de formule (I) comporte un groupe acide, la neutralisation du ou des groupes acides peut être effectuée par une base minérale, telle que LiOH, NaOH, KOH, Ca(OH)2, NH4OH, Mg(OH)2 ou Zn(OH)2; ou par une base organique telle qu'une alkylamine primaire, secondaire ou tertiaire, par exemple la triéthylamine ou la butylamine. Cette alkylamine primaire, secondaire ou tertiaire peut comporter un ou plusieurs atomes d'azote et/ou d'oxygène et peut donc comporter par exemple une ou plusieurs fonctions alcools; on peut notamment citer l'amino-2-méthyl-2-propanol, la triéthanolamine, la diméthylamino-2-propanol, le 2-amino-2-(hydroxyméthyl)-1,3-propanediol. On peut encore citer la lysine ou la 3-(diméthylamino)propylamine.

Les solvates acceptables pour les composés décrits dans la présente invention comprennent des solvates conventionnels tels que ceux formés lors de la dernière étape de préparation desdits composés du fait de la présence de solvants. A titre d'exemples, on peut citer les solvates dus à la présence d'eau ou d'alcools linéaires ou ramifiés comme l'éthanol ou l'isopropanol.

Parmi les dérivés C-glycosides de formule (I) de la composition selon l'invention, on préfère tout particulièrement :

- le C-β-D-xylopyranoside-n-propane-2-one ;
- le C-α-D-xylopyranoside-n-propane-2-one ;
- 1-phényl-2-(C-β-D-xylopyranoside)-éthane-1-one ;
- 1-phényl-2-(C-α-D-xylopyranoside)-éthane-1-one ;
- 1-[2-(3-hydroxy-propylamino)-propyl]- C-β-D-xylopyranose ;
- 1-[2-(3-hydroxy-propylamino)-propyl]- C-α-D-xylopyranose ;
- le C-β-D-xylopyranoside-2-hydroxy-propane ;
- le C-α-D-xylopyranoside-2-hydroxy-propane ;
- le C-β-D-xylopyranoside-2-amino-propane ;
- le C-α-D-xylopramoside-2-amino-propane ;
- le C-β-D-xylopyranoside-2-phénylamino-propane ;
- le C-β-D-xylopyranoside-2-phénylamino-propane ;
- l'ester éthylique de l'acide 3-méthyl-4-(C-β-D-xylopyranoside)-butyrique ;
- l'ester éthylique de l'acide 3-méthyl-4-(C-α-D-xylopyranoside)-butyrique ;
- l'acide 6-(C-β-D-xylopyranoside)-5-céto-hexanoique ;
- l'acide 6-(C-α-D-xylopyranoside)-5-céto-hexanoique ;
- l'acide 6-(C-β-D-xylopyranoside)-5-hydroxy-hexanoique ;
- l'acide 6-(C-α-D-xylopyranoside)-5-hydroxy-hexanoique ;
- l'acide 6-(C-β-D-xylopyranoside)-5-amino-hexanoique ;
- l'acide 6-(C-α-D-xylopyranoside)-5-amino-hexanoique ;
- l'acide 6-(C-β-D-xylopyranoside)-5-phenylamino-hexanoique ;
- l'acide 6-(C-β-D-xylopyranoside)-5-phenylamino-hexanoique ;
- le 1-(C-β-D-xylopyranoside)-hexane-2,6-diol ;
- le 1-(C-α-D-xylopyranoside)-hexane-2,6-diol ;
- l'acide 5-(C-β-D-xylopyranoside)-4-céto-pentanoique ;
- l'acide 5-(C-α-D-xylopyranoside)-4-céto-pentanoique ;
- l'acide 5-(C-β-D-xylopyranoside)-4-hydroxy-pentanoique ;
- l'acide 5-(C-α-D-xylopyranoside)-4-hydroxy-pentanoique ;
- l'acide 5-(C-β-D-xylopyranoside)-4-amino-pentanoique ;
- l'acide 5-(C-α-D-xylopyranoside)-4-amino-pentanoique ;
- l'acide 5-(C-β-D-xylopyranoside)-4-phénylamino-pentanoique ;
- l'acide 5-(C-α-D-xylopyranoside)-4-phénylamino-pentanoique ;
- le 1-(C-β-D-xylopyranoside)-pentane-2,5-diol ;
- le 1-(C-α-D-xylopyranoside)-pentane-2,5-diol ;
- la 1-(C-β-D-fucopyranoside)-propane-2-one ;
- la 1-(C-α-D-fucopyranoside)-propane-2-one ;
- la 1-(C-β-D-fucopyranoside)-propane-2-one ;
- la 1-(C-α-D-fucopyranoside)-propane-2-one ;
- le 1-(C-β-D-fucopyranoside)-2-hydroxy-propane ;
- le 1-(C-α-D-fucopyranoside)-2-hydroxy-propane ;
- le 1-(C-β-L-fucopyranoside)-2-hydroxy-propane ;
- le 1-(C-α-D-fucopyranoside)-2-hydroxy-propane ;
- le 1-(C-β-D-fucopyranoside)-2-amino-propane ;
- le 1-(C-α-D-fucopyranoside)-2-amino-propane ;
- le 1-(C-β-L-fucopyranoside)-2-amino-propane ;
- le 1-(C-α-L-fucopyranoside)-2-amino-propane ;
- le 1-(C-β-D-fucopyranoside)-2-phénylamino-propane ;

- le 1-(C-α-D-fucopyranoside)-2-phénylamino-propane ;
- le 1-(C-β-L-fucopyranoside)-2-phénylamino-propane ;
- le 1-(C-α-L-fucopyranoside)-2-phénylamino-propane ;
- l'ester éthylique de l'acide 3-méthyl-4-(C-β-D-fucopyranoside)-butyrique ;
- l'ester éthylique de l'acide 3-méthyl-4-(C-α-D-fucopyranoside)-butyrique ;
- l'ester éthylique de l'acide 3-méthyt-4-(C-β-L-fucopyranoside)-butyrique ;
- l'ester éthylique de l'acide 3-méthyl-4-(C-α-L-fucopyranoside)-butyrique ;
- l'acide 6-(C-β-D-fucopyranoside)-5-céto-hexanoique ;
- l'acide 6-(C-α-D-fucopyranoside)-5-céto-hexanoique ;
- l'acide 6-(C-β-L-fucopyranoside)-5-céto-hexanoique ;
- l'acide 6-(C-α-L-fucopyranoside)-5-céto-hexanoique ;
- l'acide 6-(C-β-D-fucopyranoside)-5-hydroxy-hexanoique ;
- l'acide 6-(C-α-D-fucopyranoside)-5-hydroxy-hexanoique ;
- l'acide 6-(C-β-L-fucopyranoside)-5-hydroxy-hexanoique ;
- l'acide 6-(C-α-L-fucopyranoside)-5-hydroxy-hexanoique ;
- l'acide 6-(C-β-D-fucopyranoside)-5-amino-hexanoique ;
- l'acide 6-(C-α-D-fucopyranoside)-5-amino-hexanoique ;
- l'acide 6-(C-β-L-fucopyranoside)-5-amino-hexanoique ;
- l'acide 6-(C-α-L-fucopyranoside)-5-amino-hexanoique ;
- le 1-(C-β-D-fucopyranoside)-hexane-2,6-diol ;
- le 1-(C-α-D-fucopyranoside)-hexane-2, 6-diol ;
- le 1-(C-β-L-fucopyranoside)-hexane-2,6-diol ;
- le 1-(C-α-D-fucopyranoside)-hexane-2,6-diol ;
- l'acide 5-(C-β-D-fucopyranoside)-4-céto-pentanoique ;
- l'acide 5-(C-α-D-fucopyranoside)-4-céto-pentanoique ;
- l'acide 5-(C-β-L-fucopyranoside)-hexane-2,6-diol)-4-céto-pentanoique ;
- l'acide 5-(C-α-L-fucopyranoside)-hexane-2,6-diol)-4-céto-pentanoique ;
- l'acide 5-(C-β-D-fucopyranoside)-4-hydroxy-pentanoique ;
- l'acide 5-(C-α-D-fucopyranoside)-4-hydroxy-pentanoique ;
- l'acide 5-(C-β-L-fucopyranoside)-4-hydroxy-pentanoique ;
- l'acide 5-(C-α-L-fucopyranoside)-4-hydroxy-pentanoique ;
- l'acide 5-(C-β-D-fucopyranoside)-4-annino-pentanoique ;
- l'acide 5-(C-α-D-fucopyranoside)-4-amino-pentanoique ;
- l'acide 5-(C-β-L-fucopyranoside)-4-amino-pentanoique ;
- l'acide 5-(C-α-L-fucopyranoside)-4-amino-pentanoique ;
- le 1-(C-β-D-fucopyranoside)-pentane-2,5-diol ;
- le 1-(C-a-D-fucopyranoside)-pentane-2, 5-diol ;
- le 1-(C-β-L-fucopyranoside)-pentane-2,5-diol ;
- le 1-(C-α-L-fucopyranoside)-pentane-2, 5-diol ;
- le 1-(C-β-D-Glucopyranosyl)-2-hydroxyl-propane ;
- le 1-(C-α-Glucopyranosyl)-2-hydroxyl-propane ;
- le 1-(C-β-D-Glucopyranosyl)-amino-propane ;
- le 1-(C-α-D-Glucopyranosyl)-2-amino-propane ;
- le 1-(C-β-D-Glucopyranosyl)-2-phénylamino-propane ;
- le 1-(C-α-D-Glucopyranosyl)-2-phénylamino-propane ;
- l'ester éthylique de l'acide 3-méthyl-4-(C-β-D-Glucopyranosyl)-butyrique ;
- l'ester éthylique de l'acide 3-méthyl-4-(C-α-D-Glucopyranosyl)-butyrique ;
- l'acide 6-(C-β-D-Glucopyranosyl)-5-céto-hexanoique ;
- l'acide 6-(C-α-D-Glucopyranosyl)-5-céto-hexanoique ;
- l'acide 6-(C-β-D-Glucopyranosyl)-5-hydroxy-hexanoique ;
- l'acide 6-(C-α-D-Glucopyranosyl)-5-hydroxy-hexanoique ;
- l'acide 6-(C-β-D-Glucopyranosyl)-5-amino-hexanoique ;
- l'acide 6-(C-α-D-Glucopyranosyl)-5-amino-hexanoique ;
- l'acide 6-(C-β-D-Glucopyranosyl)-5-phénylamino-hexanoique ;
- l'acide 6-(C-α-D-Glucopyranosyl)-5-phénylamino-hexanoique ;
- le 1-(C-β-D-Glucopyranosyl)-hexane-2,6-diol ;
- le 1-(C-α-D-Glucopyranosyl)-hexane-2,6-diol ;
- l'acide 6-(C-β-D-Glucopyranosyl)-5-céto-pentanoique ;

- l'acide 6-(C-α-D-Glucopyranosyl)-5-céto-pentanoique ;
- l'acide 6-(C-β-D-Glucopyranosyl)-5-hydroxy-pentanoique ;
- l'acide 6-(C-α-D-Glucopyranosyl)-5-hydroxy-pentanoique ;
- l'acide 6-(C-β-D-Glucopyranosyl)-5-amino-pentanoique ;
- l'acide 6-(C-α-D-Glucopyranosyl)-5-hydroxy-pentanoique ;
- l'acide 6-(C-β-D-Glucopyranosyl)-5-phénylamino-pentanoique ;
- l'acide 6-(C-α-D-Glucopyranosyl)-5-phénylamino-pentanique ;
- le 1-(C-β-D-Glucopyranosyl)-pentane-2,6-diol ;
- le 1-(C-α-D-Glucopyranosyl)-pentane-2, 6-diol ;
- le 1-(C-β-D-galactopyranosyl)-2-hydroxyl-propane ;
- le 1-(C-α-D-galactopyranosyl)-2-hydroxyl-propane ;
- le 1-(C-β-D-galactopyranosyl)-2-amino-propane ;
- le 1-(C-α-D-galactopyranosyl)-2-amino-propane ;
- le 1-(C-β-D-galactopyranosyl)-2-phénylamino-propane ;
- le 1-(C-α-D-galactopyranosyl)-2-phénylamino-propane ;
- l'ester éthylique de l'acide 3-méthyl-4-(β-D-galactopyranosyl)-butyrique ;
- l'ester éthylique de l'acide 3-méthyl-4-(α-D-galactopyranosyl)-butyrique ;
- l'acide 6-(C-β-D-galactopyranosyl)-5-céto-hexanoique ;
- l'acide 6-(C-α-D-galactopyranosyl)-5-céto-hexanoique ;
- l'acide 6-(C-β-D-galactopyranosyl)-5-hydro-hexanoique ;
- l'acide 6-(C-α-D-galactopyranosyl)-5-hydroxy-hexanoique ;
- l'acide 6-(C-β-D-galactopyranosyl)-5-amino-hexanoique ;
- l'acide 6-(C-α-D-galactopyranosyl)-5-amino-hexanoique ;
- l'acide 6-(C-β-D-galactopyranosyl) 5-phénylamino-hexanoique ;
- l'acide 6-(C-α-D-galactopyranosyl) 5-phénylamino-hexanoique ;
- le 1-(C-β-D-galactopyranosyl)-hexane-2,6-diol ;
- le 1-(C-α-D-galactopyranosyl)-hexane-2,6-diol ;
- l'acide 6-(C-β-D-galactopyranosyl)-5-céto-pentanoique ;
- l'acide 6-(C-α-D-galactopyranosyl)-5-céto-pentanoique ;
- l'acide 6-(C-β-D-galactopyranosyl)-5-hydroxy-pentanoique ;
- l'acide 6-(C-α-D-galactopyranosyl)-5-hydroxy-pentanoique ;
- l'acide 6-(C-β-D-galactopyranosyl)-5-amino-pentanoique ;
- l'acide 6-(C-α-D-galactopyranosyl)-5-amino-pentanoique ;
- l'acide 6-(C-β-D-galactopyranosyl)-5-phénylamino-pentanoique ;
- l'acide 6-(C-α-D-galactopyranosyl)-5-phénylamino-pentanoique ;
- le 1-(C-β-D-galactopyranosyl)-pentane-2,6-diol ;
- le 1-(C-α-D-galactopyranosyl)-pentane-2,6-diol ;
- la 1-(C-β-D-fucofuranosyl)-propane-2-one ;
- la 1-(C-α-D-fucofuranosyl)-propane-2-one ;
- la 1-(C-β-L-fucofuranosyl)-propane-2-one ;
- la 1-(C-α-L-fucofuranosyl)-propane-2-one ;
- la 3'-(acétamido-C-β-D-glucopyranosyl)-propane-2'-one ;
- la 3'-(acétamido-C-α-D-glucopyranosyl)-propane-2'-one ;
- la 1-(C-β-D-galactopyranosyl)-2-hydroxyl-propane ;
- la 1-(C-α-D-galactopyranosyl)-2-amino-propane ;
- la 1-(acétamido-C-β-D-glucopyranosyl)-2-phénylamino-propane ;
- la 1-(acétamido-C-α-D-glucopyrnaosyl)-2-phénylamino-propane ;
- l'ester éthylique de l'acide 3-méthyl-4-(acétamido-C-β-D-glucopyranosyl) butyrique ;
- l'ester éthylique de l'acide 3-méthyl-4-(acétamido-C-α-D-glucopyranosyl) butyrique ;
- l'acide 6-(acétamido-C-β-D-glucopyranosyl)-5-céto-hexanoique ;
- l'acide 6-(acétamido-C-α-D-glucopyranosyl)-5-céto-hexanoique ;
- l'acide 6-(acétamido-C-β-D-glucopyranosyl)-5-hydroxy-hexanoique ;
- l'acide 6-(acétamido-C-α-D-glucopyranosyl)-5-hydroxy-hexanoique ;
- l'acide 6-(acétamido-C-β-D-glucopyranosyl)-5-amino-hexanoique ;
- l'acide 6-(acétamido-C-α-D-glucopyranosyl)-5-amino-hexanoique ;
- l'acide 6-(acétamido-C-β-D-glucopyranosyl) 5-phénylamino-hexanoique ;
- l'acide 6-(acétamido-C-α-D-glucopyranosyl)-5-phénylamino-hexanoique ;
- la 1-(acétamido-C-β-D-glucopyranosyl)-hexane-2,6-diol ;

- la 1-(acétamido-C-α-D-glucopyranosyl)-hexane-2,6-diol ;
- l'acide 6-(acétamido-C-β-D-glucopyranosyl)-5-céto-pentanoique ;
- l'acide 6-(acétamido-C-α-D-glucopyranosyl)-5-céto-pentanoique ;
- l'acide 6-(acétamido-C-β-D-glucopyranosyl)-5-hydroxy-pentanoique ;
- l'acide 6-(acétamido-C-α-D-glucopyranosyl)-5-hydroxy- pentanoique ;
- l'acide 6-(acétamido-C-β-D-glucopyranosyl)-5-amino-pentanoique ;
- l'acide 6-(acétamido-C-α-D-glucopyranosyl)-5-amino-pentanoique ;
- l'acide 6-(acétamido-C-β-D-glucopyranosyl)-5-phénylamino- pentanoique ;
- l'acide 6-(acétamido-C-α-D-glucopyranosyl)-5-phénylamino- pentanoique ;
- la 1-(acétamido-C-β-D-glucopyranosyl)-pentane-2,6-diol ;
- la 1-(acétamido-C-α-D-glucopyranosyl)-pentane-2,6-diol.

A titre illustratif et non limitatif des dérivés C-glycosides convenant plus particulièrement à l'invention, on peut notamment citer les dérivés suivants :

le C-[beta]-D-xylopyranoside-n-propane-2-one, le C-[alpha]- D-xylopyranoside-n-propane-2-one, le C-[beta]-D-xylopyranoside-2-hydroxy-propane, le C-[alpha]- D-xylopyranoside-2-hydroxy-propane, la 1-(C-[beta]-D-fucopyranoside)-propane-2-one, la 1-(C-[alpha]-D-fucopyranoside)-propane-2-one, la 1-(C-[beta]-L-fucopyranoside)-propane-2-one, la 1-(C-[alpha]-L-fucopyranoside)-propane-2-one, le 1-(C-[beta]-D-fucopyranoside)-2-hydroxy-propane, le 1-(C-[alpha]-D-focopyranoside)-2-hydroxy-propane, le 1-(C-[beta]-L-fucopyranoside)-2-hydroxy-propane, le 1-(C-[alpha]-L-fucopyranoside) -2-hydroxy-propane, le 1-(C-[beta]-D-glucopyranosyl)-2-hydroxyl-propane, le 1-(C-[alpha]-D-glucopyranosyl)-2-hydroxyl-propane, le 1-(C-[beta]-D-galactopyranosyl)-2-hydroxyl-propane, le 1-(C-[alpha]-D-galactopyranosyl)-2-hydroxyl-propane, la 1-(C-[beta]-D-fucofuranosyl)-propane-2-one, la 1-(C-[alpha]-D-fucofuranosyl)-propane-2-one, la 1-(C-[beta]-L-fucofuranosyl)-propane-2-one, la 1-(C-[alpha]-L-fucofuranosyl)-propane-2-one, le C-[beta]-D-maltopyranoside-n-propane-2-one, le C-[alpha]-D-maltopyranoside-n-propane-2-one, le C-[beta]-D-maltopyranoside-2-hydroxy-propane, le C-[alpha]-D-maltopyranoside-2-hydroxy-propane, leurs isomères et leurs mélanges.

Selon un mode de réalisation, le C-[beta]-D-xylopyranoside-2-hydroxy-propane ou le C-[alpha]-D-xylopyranoside-2-hydroxy-propane, et mieux le C-[beta]-D-xylopyranoside-2-hydroxy-propane, peuvent être avantageusement mis en oeuvre pour la préparation d'une composition selon l'invention.

Selon un mode de réalisation particulier, le dérivé C-glycoside peut être le C-[beta]-D-xylopyranoside-2-hydroxy-propane sous forme d'une solution à 30 % en poids en matière active dans un mélange eau/propylène glycol (60/40 % en poids) tel que le produit fabriqué par CHIMEX sous la dénomination commerciale "MEXORYL SBB®".

Bien entendu, selon l'invention, un dérivé C-glycoside répondant à la formule (I) peut être utilisé seul ou en mélange avec d'autres dérivés C-glycosides et en toutes proportions. Un dérivé C-glycoside convenant à l'invention peut notamment être obtenu par la méthode de synthèse décrite dans le document WO 02/051828.

La présente invention porte aussi sur l'utilisation d'une composition selon l'invention telle que définie précédemment, administrée par voie orale, topique ou par injection cutanée, notamment pour le soin de la peau et/ou du cuir chevelu.

Une composition conforme à l'invention telle que définie précédemment peut notamment être une composition cosmétique pour soin capillaire pour, en particulier, la stimulation de la pousse du cheveu, la lutte contre la chute des cheveux, le ralentissement de sa chute ou le renforcement de l'éclat du cheveu.

La présente invention a également pour objet une méthode de traitement, en particulier cosmétique ou thérapeutique, pour diminuer ou prévenir les signes du vieillissement de la peau ou de ses phanères (cheveux, cils, ongles, ...), par administration à un sujet, de préférence un être humain, d'une quantité efficace d'au moins un monosaccharide tel que défini précédemment en association avec une quantité efficace d'au moins un composé additionnel tel que défini précédemment. L'invention a en particulier pour objet un procédé de traitement cosmétique d'une peau ridée, en particulier la peau du visage et/ou du front, comprenant l'application topique sur ladite peau d'une composition comprenant, dans un milieu physiologiquement acceptable, l'association d'une quantité efficace d'au moins un monosaccharide tel que défini précédemment et d'une quantité efficace d'au moins un composé additionnel tel que défini précédemment.

La présente invention concerne également l'utilisation de la composition ou de l'association selon l'invention, pour diminuer et/ou prévenir les signes du vieillissement de la peau ou de ses phanères.

La composition ou l'association selon l'invention permet aussi de stimuler la régénération des cellules de l'épiderme et du derme, au niveau de la peau ou des phanères, en particulier les kératinocytes et les fibroblastes, notamment par augmentation de leur prolifération. On dispose de la sorte d'une méthode, notamment cosmétique, efficace notamment pour lutter contre les signes du chronovieillissement et/ou du photovieillissement.

Les signes du photovieillissement correspondent aux dégradations internes de la peau consécutives à une

exposition aux rayonnements ultra-violets (vieillissement actinique). Les signes du chronovieillissement correspondent aux dégradations internes de la peau dues au vieillissement intrinsèque des individus.

Selon une forme de réalisation préférée, l'utilisation selon la présente invention est destinée à améliorer l'éclat du teint, à diminuer et/ou prévenir les caractéristiques des rides et/ou ridules, à améliorer et/ou diminuer le micro-relief de la peau, à lisser la peau et/ou à améliorer les propriétés mécaniques de la peau et/ou augmenter la résistance de la peau aux agressions mécaniques, telles que frottements, tensions, frictions et/ou favoriser la réparation de la peau.

Selon un autre aspect de l'invention, l'utilisation de la composition ou de l'association selon l'invention permet d'améliorer la densité de la peau, sa fermeté et/ou la cohésion de ses différents compartiments, en particulier la cohésion du derme avec l'épiderme.

La présente invention concerne également l'utilisation de la composition ou de l'association selon l'invention pour traiter, de manière préventive ou curative, les rides et/ou ridules, la peau flétrie, le manque d'élasticité et/ou de tonus de la peau, l'amincissement du derme, la dégradation des fibres de collagène, la peau molle, la peau amincie, et/ou les dégradations internes de la peau consécutives à une exposition aux rayonnements ultra-violets.

La composition ou l'association selon l'invention permet de stimuler la synthèse des glycosaminoglycannes contenant un résidu D-glucosamine et/ou N-acétyl-D glucosamine, avantageusement de l'acide hyaluronique, et/ou des protéoglycannes, avantageusement des protéoglycannes contenant de l'acide hyaluronique, par les fibroblastes et/ou les kératinocytes.

La composition ou l'association selon la présente invention a également pour effet d'augmenter la synthèse des laminines, de préférence la laminine V, et la synthèse des collagènes, de préférence choisis parmi le pro-collagène I, le collagène IV et le collagène VII.

La quantité des ingrédients actifs, choisis parmi les monosaccharides et les C-glycosides et dérivés précédemment définis, à mettre en oeuvre selon l'invention dépend de l'effet cosmétique ou thérapeutique recherché, et peut donc varier dans une large mesure. L'homme de l'art peut aisément, sur la base de ses connaissances générales, déterminer les quantités appropriées.

Ainsi, et selon une forme de réalisation préférée, la composition selon l'invention comprend au moins un monosaccharide tel que défini ci-dessus en une quantité comprise entre 0,001 % et 30 % en poids par rapport au poids total de la composition, et en particulier entre 0,1% et 10 % en poids, et plus particulièrement entre 0,5 % et 6 % en poids par rapport au poids total de la composition.

Selon une forme de réalisation préférée, la composition selon l'invention comprend un C-glycoside et/ou au moins un de ses dérivés en une quantité comprise entre 0,001 % et 30 % en poids par rapport au poids total de la composition, et en particulier entre 0,1% et 10 % en poids, et plus particulièrement entre 0,5 % et 6 % en poids par rapport au poids total de la composition.

En particulier, la composition selon l'invention ne comprend pas l'association de xylitol et de mannitol.

De manière particulière, la composition selon l'invention ne comprend pas d'agent tenseur.

De manière générale, on entend par « agent tenseur » tous composés solubles ou dispersibles dans l'eau à une température allant de 25°C à 50°C à la concentration de 7 % en poids dans l'eau ou à la concentration maximale à laquelle ils forment un milieu d'apparence homogène et produisant à cette concentration de 7 % ou à cette concentration maximale dans l'eau une rétractation de plus de 15 % dans le test décrit ci-après.

La concentration maximale à laquelle ils forment un milieu d'apparence homogène est déterminée à ± 20 % près et de préférence à ± 5% près.

On entend par "milieu d'apparence homogène" un milieu ne présentant pas d'agrégats visibles à l'oeil nu.

Pour la détermination de ladite concentration maximale, l'agent tenseur est ajouté progressivement dans l'eau sous agitation à la défloculeuse à une température allant de 25°C à 50°C, puis le mélange est maintenu sous agitation pendant une heure. On observe ensuite après 24 heures si le mélange ainsi préparé est d'apparence homogène (absence d'agrégats visibles à l'oeil nu).

L'effet tenseur peut être caractérisé par un test *in vitro* de rétractation.

On prépare préalablement et tel que décrit précédemment un mélange homogène de l'agent tenseur dans l'eau, à la concentration de 7 % en poids ou à la concentration maximale définie précédemment.

30μL du mélange homogène est déposé sur une éprouvette rectangulaire (10x40mm, donc présentant une largeur initiale $L_0$ de 10 mm) d'élastomère ayant un module d'élasticité de 20MPa et une épaisseur de 100μm.

Après 3h de séchage à 22±3°C et 40±10% d'humidité relative HR, l'éprouvette d'élastomère présente une largeur rétractable, notée $L_{3h}$ due à la tension exercée par l'agent tenseur déposé.

L'effet tenseur (ET) dudit agent est alors qualifié de la façon suivante :

$$'ET' = (L_0 - L_{3h} / L_0)x100 \text{ en } \%$$

| |
|---|
| avec $L_0$ = largeur initiale 10mm<br>et $L_{3h}$ = largeur après 3h de séchage |

L'agent tenseur peut être choisi parmi :

a) les protéines végétales ou animales et leurs hydrolysats ;
b) les polysaccharides d'origine naturelle ;
c) les silicates mixtes ;
d) les particules colloïdales de charges inorganiques ;
e) les polymères synthétiques ;

et les mélanges de ceux-ci.
L'homme du métier saura choisir, dans les catégories chimiques listées ci-dessus, les matériaux répondant au test tel que décrit précédemment.

La composition selon l'invention est adaptée à une administration topique sur la peau ou ses phanères, une administration orale ou une injection cutanée, en particulier sous la forme d'une solution stérile.

De préférence, les administrations topiques selon l'invention se présentent sous forme d'une crème, d'un gel, d'une lotion, d'un lait, d'une huile, d'un onguent, d'une cire, d'une mousse, d'une pâte, d'un sérum, d'une pommade ou d'un shampooing.

De manière également préférée, les administrations orales selon l'invention se présentent sous la forme d'une gélule, d'un comprimé, ou de pilules.

Le monosaccharide selon l'invention, et le C-glycoside ou un de ses dérivés, sont plus particulièrement présents dans la composition selon l'invention à titre d'agent (ou ingrédient) actif, en particulier de seuls agents actifs.

Par « agent actif » ou « ingrédient actif », on entend plus spécifiquement selon l'invention, un composé qui, lorsqu'il est administré à un sujet, en particulier un sujet humain, joue un rôle biologique direct sur l'organisme, en particulier sur la peau ou ses phanères, en particulier sans améliorer l'effet biologique ou mécanique d'un autre composé présent dans la composition selon l'invention.

D'une manière générale, le milieu dans lequel sont compris les principes actifs de la composition tel que définie précédemment, est un milieu physiologiquement acceptable, en particulier un milieu cosmétiquement ou pharmaceutiquement acceptable et peut être anhydre ou aqueux. Il peut ainsi comprendre une phase aqueuse et/ou une phase grasse.

Le milieu physiologiquement acceptable dans lequel les composés selon l'invention peuvent être employés, ainsi que ses constituants, leur quantité, la forme galénique de la composition, son mode de préparation, et son mode d'administration peuvent être choisis par l'homme du métier sur la base de ses connaissances générales en fonction du type de composition recherchée.

Lorsque la composition est une composition destinée à une administration topique, elle peut se présenter avantageusement sous la forme de solutions aqueuses, hydroalcooliques, d'émulsions huile-dans-eau (H/E) ou eau-dans-huile (E/H) ou multiple (triple : E/H/E ou H/E/H), des nanoémulsions, en particulier des nanoémulsions H/E, dont la tailles des gouttes est inférieure à 100nm, de gels aqueux, ou de dispersions d'une phase grasse dans une phase aqueuse à l'aide de sphérules, ces sphérules pouvant être des nanoparticules polymériques telles que les nanosphères et les nanocapsules ou des vésicules lipidiques de type ionique et/ou non ionique (liposomes, niosomes, oléosomes (tels que décrits dans les demandes FR2709666 et FR2725369)).

Ces compositions sont préparées selon les méthodes usuelles.

En outre, les compositions utilisables selon l'invention peuvent être plus ou moins fluides et avoir l'aspect d'une crème blanche ou colorée, d'une pommade, d'un lait, d'une lotion, d'un sérum, d'une pâte ou d'une mousse. Elles peuvent être éventuellement appliquées sur la peau sous forme d'aérosol. Elles peuvent aussi se présenter sous forme solide, et par exemple sous forme de stick.

Pour une application locale sur les cheveux ou le cuir chevelu, la composition peut être sous forme de solutions aqueuses, alcooliques ou hydroalcooliques; sous forme de crèmes, de gels, d'émulsions, de mousses; sous forme de compositions pour aérosol comprenant également un agent propulseur sous pression.

Lorsque la composition se présente sous forme aqueuse, notamment sous forme de dispersion, d'émulsion ou de solution aqueuse, elle peut comprendre une phase aqueuse, qui peut comprendre de l'eau, une eau florale et/ou une eau minérale.

Lorsque la composition est une émulsion, la proportion de la phase grasse peut varier d'environ 5 % à 80 %

en poids, et de préférence d'environ 2 % à 50 % en poids par rapport au poids total de la composition. Les huiles, les cires, les émulsionnants et les co-émulsionnants utilisés dans la composition sous forme d'émulsion sont choisis parmi ceux classiquement utilisés dans le domaine cosmétique. L'émulsionnant et le co-émulsionnant sont présents, dans la composition, en une proportion allant de 0,3 % à 30 % en poids, et de préférence de 0,5 % à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

La phase huileuse peut comprendre aussi tout additif habituel liposoluble ou lipodispersible comme indiqué ci-après.

Elle peut notamment comprendre des corps gras tels que des cires, des composés pâteux, des alcools gras, des acides gras. La phase huileuse contient au moins une huile, plus particulièrement au moins une huile cosmétique. On entend par "huile" un corps gras liquide à la température ambiante (25°C).

Comme huiles utilisables dans la composition de l'invention, on peut citer par exemple :

- les huiles hydrocarbonées d'origine animale, telles que le perhydrosqualène;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple, les huiles de tournesol, de maïs, de soja, de courge, de pépins de raisin, de sésame, de noisette, d'abricot, de macadamia, d'arara, de coriandre, de ricin, d'avocat, les triglycérides des acides caprylique/caprique comme ceux commercialisés par la société Stearineries Dubois ou ceux commercialisés sous les dénominations Miglyol 810, 812 et 818 par la société Dynamit Nobel, l'huile de jojoba, l'huile de beurre de karité, le caprylyl glycol ;
- les esters et les éthers de synthèse, notamment d'acides gras, comme les huiles de formules R1 COOR2 et R1OR2 dans laquelle R1 représente le reste d'un acide gras ou d'un alcool gras comportant de 8 à 29 atomes de carbone, et R2 représente une chaîne hydrocarbonée, ramifiée ou non, contenant de 3 à 30 atomes de carbone, comme par exemple l'huile de Purcellin, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrytyle, l'isopropyl lauroyl sarcosinate, notamment vendu sous le nom commercial Eldew SL 205 de la société Ajinomoto ;
- les hydrocarbures linéaires ou ramifiés, d'origine minérale ou synthétique, tels que les huiles de paraffine, volatiles ou non, et leurs dérivés, la vaseline, les polydécènes, l'isohexadecane, l'isododecane, le polyisobutène hydrogéné tel que l'huile de Parléam, le mélange de n-undécane (C11) et de n-tridécane (C13) commercialisé sous la référence de CETIOL UT par la Société Cognis ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les huiles de silicone volatiles, en particulier cyclopolydiméthylsiloxanes (cyclométhicones) telles que le cyclohexadiméthylsiloxane et le cyclopentadiméthylsiloxane ; lespolydiméthyl-siloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les résines de silicone telles que la trifluorométhyl-C1-4-alkyldimethicone et la trifluoropropyldimethicone ; et les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries.

Ces corps gras peuvent être choisis de manière variée par l'homme du métier afin de préparer une composition

ayant les propriétés, par exemple de consistance ou de texture, souhaitées.

Les émulsions contiennent généralement au moins un émulsionnant choisi parmi les émulsionnants amphotères, anioniques, cationiques ou non ioniques, utilisés seuls ou en mélange, et éventuellement un co-émulsionnant. Les émulsionnants sont choisis de manière appropriée suivant l'émulsion à obtenir (E/H ou H/E). L'émulsionnant et le co-émulsionnant sont généralement présents dans la composition, en une proportion allant de 0,3 à 30 % en poids, et de préférence de 0,5 à 20 % en poids par rapport au poids total de la composition.

Pour les émulsions E/H, on peut citer par exemple comme émulsionnants les dimethicone copolyols tels que le mélange de cyclomethicone et de dimethicone copolyol, vendu sous la dénomination « DC 5225 C » par la société Dow Corning, et les alkyl-dimethicone copolyols tels que le Laurylmethicone copolyol vendu sous la dénomination « Dow Corning 5200 Formulation Aid » par la société Dow Corning et le Cetyl dimethicone copolyol vendu sous la dénomination « Abil EM 90® » par la société Goldschmidt. On peut aussi utiliser comme tensioactif d'émulsions E/H, un organopolysiloxane solide élastomère réticulé comportant au moins un groupement oxyalkyléné, tel que ceux obtenus selon le mode opératoire des exemples 3, 4 et 8 du document US-A-5,412,004 et des exemples du document US-A-5,811,487, notamment le produit de l'exemple 3 (exemple de synthèse) du brevet US-A-5,412,004. et tel que celui commercialisé sous la référence KSG 21 par la société Shin Etsu.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose ; et leurs mélanges tels que le mélange de stéarate de glycéryle et de stéarate de PEG-40.

Ces compositions peuvent être également des émulsions H/E stabilisées par des particules comme par exemple des particules polymériques décrites dans le brevet FR2760641, des polymères amphiphiles réticulés ou non tels que décrits dans les demandes : FR2853543 et FR2819175.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les absorbeurs d'odeurs et les matières colorantes. Les quantités de ces différents adjuvants sont celles classiquement utilisées dans le domaine cosmétique, et par exemple varient d'environ 0,01 % à 10 % du poids total de la composition. Ces adjuvants, selon leur nature, peuvent être introduits dans la phase grasse, dans la phase aqueuse et/ou dans les sphérules lipidiques.

Comme solvants utilisables dans l'invention, on peut citer les alcools inférieurs notamment l'éthanol, l'isopropanol, le dipropylène glycol, le butylène glycol et le propylène glycol.

Comme gélifiants hydrophiles utilisables dans l'invention, on peut citer à titre d'exemples non limitatifs, les polymères carboxyvinyliques (carbomer®), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium, la silice hydrophobe, l'éthylcellulose et le polyéthylène.

Lorsque la composition est administrée par voie orale, elle est avantageusement sous la forme d'une gélule, d'un comprimé, ou de pilules. Lorsque la composition est administrée par injection cutanée, elle est en particulier sous la forme d'une solution stérile.

Les compositions de l'invention peuvent contenir d'autres actifs hydrophiles ou lipophiles. Ces actifs sont notamment choisis parmi les agents anti-oxydants, les agents dermo-relaxants ou dermodécontractants, les agents anti-âge, les agents anti-glycation, les agents stimulants la synthèse de macromolécules dermiques ou épidermiques et/ou empêchant leur dégradation, les agents stimulants la prolifération des fibroblastes ou des kératinocytes et/ou la différenciation des kératinocytes, les agents favorisant la maturation de l'enveloppe cornée, les inhibiteurs de NO synthase et les agents stimulant le métabolisme énergétique des cellules. Des listes de ces actifs sont données à la suite à titre illustratif, et ne doivent en aucune façon être considérées comme limitatives.

Agents anti-âge :

Parmi les actifs connus pour lutter contre les signes du vieillissement, notamment cutané, on peut citer notamment :

la vitamine B3, le coenzyme Q10 (ou ubiquinone), la vitamine B9, la vitamine E, les dérivés de la vitamine E tels que le dérivé phosphaté comme par exemple le TPNA® commercialisé par la société Showa Denko, le resvératrol ou ses dérivés, comme par exemple le resveratrate® commercialisé par la société Estée Lauder, le rétinol ou ses dérivés, et leur mélange.

Agents anti-glycation:

Par « agent anti-glycation », on entend un composé prévenant et/ou diminuant la glycation des protéines de la peau, en particulier des protéines du derme, telles que le collagène.

Comme agents anti-glycation, on peut citer notamment les extraits végétaux de la famille des *Ericaceae,* tels qu'un extrait de myrtille (*Vaccinium angusfifollium, Vaccinium myrtillus*), par exemple celui vendu sous la dénomination « BLUEBERRY HERBASOL EXTRACT PG » par la société COSMETOCHEM, l'ergothionéine et ses dérivés, les hydroxystilbènes et leurs dérivés, tels que le resvératrol et le 3,3', 5,5'-tétrahydroxystilbène (ces agents anti-glycation sont décrits dans les demandes FR 2 802 425, FR 2 810 548, FR 2 796 278 et FR 2 802 420, respectivement), les dihydroxystilbènes et leurs dérivés, les polypeptides d'arginine et de lysine tels que celui vendu sous la dénomination « AMADORINE® » par la société SOLABIA, le chorhydrate de carcinine (commercialisé par Exsymol sous la dénomination « ALISTIN®»), un extrait *d'Hélianthus annuus* comme l'Antiglyskin® de SILAB, les extraits de vin tel que l'extrait de vin blanc en poudre sur support maltodextrine vendu sous la dénomination « Vin blanc déshydraté 2F » par la société Givaudan, l'acide thioctique (ou acide alpha lipoïque), un mélange d'extrait de busserole et de glycogène marin comme l'Aglycal LS 8777® de Laboratoires Sériobiologiques, un extrait de thé noir comme le Kombuchka® de Sederma et leurs mélanges.

Comme agents anti-glycation préférés, on citera les extraits de myrtille *(Vaccinium myrtillus)* et l'extrait de thé noir.

Agents stimulant la synthèse de macromolécules dermiques et/ou épidermiques et/ou empêchant leur dégradation :

Parmi les actifs stimulant les macromolécules du derme ou empêchant leur dégradation, on peut citer ceux qui agissent :

- soit sur la synthèse du collagène, tels que les extraits de Centella asiatica, les asiaticosides et dérivés ; les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine® ; les peptides de riz tel que le Nutripeptide® de SILAB, le méthylsilanol mannuronate tel que l'Algisium C® commercialisé par Exsymol ; les hormones végétales telles que les auxines et les lignanes ; l'acide folique ; et un extrait de Medicago sativa (alfafa) tel que celui commercialisé par SILAB sous la dénomination Vitanol®; un extrait peptidique de noisette tel que celui commercialisé par la société Solabia sous la dénomination Nuteline C®; et l'arginine.

- soit sur l'inhibition de la dégradation du collagène, en particulier des agents agissant sur l'inhibition des métalloprotéinases (MMP) telles que plus particulièrement les MMP 1, 2, 3, 9 . On peut citer : les rétinoïdes et dérivés, les extraits de medicago sativa tels que le Vitanol® de Silab, un extrait d'aphanizomenon flos-aquae (cyanophycée) commercialisée sous la dénomination Lanablue® par Atrium Biotechnologies, les oligopeptides et les lipopeptides, les lipoaminoacides, l'extrait de malt commercialisé par la société COLETICA sous la dénomination commerciale Collalift® ; les extraits de myrtille ou de romarin ; le lycopène ; les isoflavones, leurs dérivés ou les extraits végétaux en contenant, en particulier les extraits de soja (commercialisé par exemple par la société ICHIMARU PHARCOS sous la dénomination commerciale Flavostérone SB®), de trèfle rouge, de lin, de kakkon; un extrait de litchi ; la DIPALMITOYL HYDROXYPROLINE commercialisé par Seppic sous le nom SEPILIFT DPHP® : Baccharis genistelloide ou Baccharine commercialisé par SILAB, un extrait de moringa tel que l'Arganyl LS 9781® de Cognis ; l'extrait de sauge décrit dans la demande FR-A-2812544 de la famille des labiées *(salvia officinalis* de la société Flacksmann), l'extrait de Rhododendron, le blueberry extract, un extrait de *vaccinium myrtillus* tel que ceux décrits dans la demande FR-A-2814950.

- soit sur la synthèse de molécules appartenant à la famille des élastines (élastine et fibrilline), tels que : le rétinol et dérivés en particulier le palmitate de rétinol ; l'extrait de *Saccharomyces Cerivisiae* commercialisé par la société LSN sous la dénomination commerciale Cytovitin® ; et l'extrait d'algue *Macrocystis pyrifera* commercialisé par la société SECMA sous la dénomination commerciale Kelpadelie®; un extrait peptidique de noisette tel que celui commercialisé par la société Solabia sous la dénomination Nuteline C®.

- soit sur l'inhibition de la dégradation de l'élastine tels que l'extrait peptidique de graines de *Pisum sativum* commercialisé par la société LSN sous la dénomination commerciale Parelastyl® ; les héparinoïdes ; et les composés N-acylaminoamides décrits dans la demande WO 01/94381 tels que l'acide {2-[acétyl-(3-trifluorométhyl-phényl)-amino]-3-méthyl-butyrylamino} acétique, autrement nommé N-[N-acétyl, N'-(3-trifluorométhyl)phényl valyl]glycine ou N-acétyl-N-[3-(trifluorométhyl)phényl]valyl-glycine ou acétyl trifluorométhyl phényl valylglycine, ou un ester de celui-ci avec un alcool en $C_1$-$C_6$. ; un extrait de peptides de riz tel que la Colhibin® de Pentapharm, ou un extrait de Phyllanthus emblica tel que l'Emblica® de Rona.

- soit sur la synthèse des glycosaminoglycannes, tels que le produit de fermentation du lait par lactobacillus vulgaris, commercialisé par la société BROOKS sous la dénomination commerciale Biomin yogourth® ; l'extrait d'algue brune Padina pavonica commercialisé par la société ALBAN MÜLLER sous la dénomination commerciale HSP3® ; l'extrait de *Saccharomyces cerevisiae* disponible notamment auprès de la société SILAB sous la dénomination commerciale Firmalift® ou auprès de la société LSN sous la dénomination commerciale Cytovitin® ; un extrait de *Laminaria ochroleuca* tel que la Laminaïne® de Secma ; l'essence de Mamaku de Lucas Meyer, un extrait de cresson (Odraline® de Silab).
- soit sur la synthèse de la fibronectine, tels que l'extrait de zooplancton Salina commercialisé par la société SEPORGA sous la dénomination commerciale GP4G®;

l'extrait de levure disponible notamment auprès de la société ALBAN MÜLLER sous la dénomination commerciale Drieline® ; et le palmitoyl pentapeptide commercialisé par la société SEDERMA sous la dénomination commerciale Matrixil®.

Parmi les actifs stimulant les macromolécules épidermiques, telles que la fillagrine et les kératines, on peut citer notamment l'extrait de lupin commercialisé par la société SILAB sous la dénomination commerciale Structurine® ; l'extrait de bourgeons de hêtre *Fagus sylvatica* commercialisé par la société GATTEFOSSE sous la dénomination commerciale Gatuline® RC; et l'extrait de zooplancton Salina commercialisé par la société SEPORGA sous la dénomination commerciale GP4G®; Tripeptide de Cuivre de PROCYTE ; un extrait peptidique de *Voandzeia substerranea* tel que celui commercialisé par la société Laboratoires Sérobiologiques sous la dénomination commerciale Filladyn LS 9397®.

De préférence, on utilisera un actif stimulant la synthèse de macromolécules dermiques et/ou épidermiques et/ou empêchant leur dégradation choisi parmi les agents stimulant la synthèse des glycosaminoglycanes, les agents inhibant la dégradation de l'élastine, les agents stimulant la synthèse de la fibronectine, les agents stimulant la synthèse de macromolécules épidermiques, et leurs mélanges.

Encore plus préférentiellement, on utilisera un actif stimulant la synthèse des glycosaminoglycanes choisi parmi un extrait d'algue brune Padina pavonica, un extrait de *Saccharomyces cerevisiae,* un extrait de *Laminaria ochroleuca,* l'essence de Mamaku, un extrait de cresson et leurs mélanges.

Comme actifs préférés stimulant la synthèse de macromolécules dermiques et/ou épidermiques et/ou empêchant leur dégradation, on peut citer :

les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine® ; les peptides de riz tel que le Nutripeptide® de SILAB, le méthylsilanol mannuronate tel que l'Algisium C® commercialisé par Exsymol ; l'acide folique ; un extrait de Medicago sativa (alfafa) tel que celui commercialisé par SILAB sous la dénomination Vitanol®; un extrait peptidique de noisette tel que celui commercialisé par la société Solabia sous la dénomination Nuteline C®; l'arginine ; un extrait d'aphanizomenon flos-aquae (cyanophycée) commercialisée sous la dénomination Lanablue® par Atrium Biotechnologies, l'extrait de malt commercialisé par la société COLETICA sous la dénomination commerciale Collalift® le lycopène ; un extrait de litchi ; un extrait de moringa tel que l'Arganyl LS 9781® de Cognis ; un extrait de *vaccinium myrtillus* tel que ceux décrits dans la demande FR-A-2814950 ; le retinol et dérivés en particulier le palmitate de rétinol ; l'extrait de *Saccharomyces Cerivisiae* commercialisé par la société LSN sous la dénomination commerciale Cytovitin® ;
un extrait peptidique de noisette tel que celui commercialisé par la société Solabia sous la dénomination Nuteline C® ; l'acide {2-[acétyl-(3-trifluorométhyl-phényl)-amino]-3-méthyl-butyrylamino} acétique, autrement nommé N-[N-acétyl, N'-(3-trifluorométhyl)phényl valyl]glycine ou N-acetyl-N-[3-(trifluoromethyl)phenyl]valylglycine ou acetyl trifluoromethyl phenyl valylglycine, ou un ester de celui-ci avec un alcool en $C_1$-$C_6$.; un extrait de peptides de riz tel que la Colhibin® de Pentapharm, ou un extrait de Phyllanthus emblica tel que l'Emblica® de Rona ;
l'extrait d'algue brune Padina pavonica commercialisé par la société ALBAN MÜLLER sous la dénomination commerciale HSP3® ; l'extrait de *Saccharomyces cerevisiae* disponible notamment auprès de la société SILAB sous la dénomination commerciale Firmalift® ou auprès de la société LSN sous la dénomination commerciale Cytovitin® ; un extrait de *Laminaria ochroleuca* tel que la Laminaïne® de Secma ; l'essence de Mamaku de Lucas Meyer, l'extrait de lupin commercialisé par la société SILAB sous la dénomination commerciale Structurine® ; l'extrait de bourgeons de hêtre *Fagus sylvatica* commercialisé par la société GATTEFOSSE sous la dénomination commerciale Gatuline® RC.

Agents stimulant la prolifération des fibroblastes ou des kératinocytes et/ou la différenciation des kératinocytes

Les agents stimulant la prolifération des fibroblastes utilisables dans la composition selon l'invention peuvent par exemple être choisis parmi les protéines ou polypeptides végétaux, extraits notamment du soja (par exemple un extrait de soja commercialisé par la société LSN sous la dénomination Eleseryl SH-VEG 8® ou commercialisé par la société SILAB sous la dénomination commerciale Raffermine®) ; un extrait de protéines hydrolysées de soja tel que le RIDULISSE® de SILAB ; et les hormones végétales telles que les giberrellines et les cytokinines ; un extrait peptidique de noisette tel que celui commercialisé par la société Solabia sous la dénomination Nuteline C®.

De préférence on utilisera un agent favorisant la prolifération et/ou la différenciation des kératinocytes.

Les agents stimulant la prolifération des kératinocytes, utilisables dans la composition selon l'invention, comprennent notamment ; le phloroglucinol, l'extrait de feuille d'hydrangea macrophylla comme l'Amacha liquid E® d'Ichimaru Pharcos, un extrait de levure tel que le Stimoderm® de CLR ; l'extrait de *Larrea divaricata* tel que le Capislow® de Sederma, les mélanges d'extraits de papaye, de feuilles d'olivier et de citron tel que la Xyléine® de Vincience, l'extrait de feuille d'hydrangea macrophylla comme l'Amacha liquid E® d'Ichimaru Pharcos, le rétinol et ses esters dont le palmitate de rétinyle, les extraits de tourteaux de noix commercialisés par Gattefosse et les extraits de *solanum tuberosum* tel que le Dermolectine® commercialisé par Sederma.

Parmi les agents stimulant la différenciation des kératinocytes comprennent par exemple les minéraux tels que le calcium; un extrait peptidique de lupin tel que celui commercialisé par la société SILAB sous la dénomination commerciale Structurine®; le beta-sitosteryl sulfate de sodium tel que celui commercialisé par la société SEPORGA sous la dénomination commerciale Phytocohésine® ; et un extrait hydrosoluble de maïs tel que celui commercialisé par la société SOLABIA sous la dénomination commerciale Phytovityl® ; un extrait peptidique de *Voandzeia substerranea* tel que celui commercialisé par la société Laboratoires Sérobiologiques sous la dénomination commerciale Filladyn LS 9397® ; et les lignanes tels que le sécoisolaricirésinol, le rétinol et ses esters dont le palmitate de rétinyle.

Comme agents stimulant la prolifération et/ou la différenciation des kératinocytes, on peut citer encore les oestrogènes tel que l'oestradiol et homologues ; les cytokines.

Comme actifs stimulant la prolifération des fibroblastes ou des kératinocytes et/ou la différenciation des kératinocytes préférés, on citera des protéines ou polypeptides végétaux, extraits notamment du soja (par exemple un extrait de soja commercialisé par la société LSN sous la dénomination Eleseryl SH-VEG 8® ou commercialisé par la société SILAB sous la dénomination commerciale Raffermine®) ; un extrait de protéines hydrolysées de soja tel que le RIDULISSE® de SILAB ; un extrait peptidique de noisette tel que celui commercialisé par la société Solabia sous la dénomination Nuteline C® ; l'adénosine, le phloroglucinol, un extrait de levure tel que le Stimoderm® de CLR ; un extrait peptidique de lupin tel que celui commercialisé par la société SILAB sous la dénomination commerciale Structurine®; un extrait hydrosoluble de maïs tel que celui commercialisé par la société SOLABIA sous la dénomination commerciale Phytovityl® ; un extrait peptidique de *Voandzeia substerranea* tel que celui commercialisé par la société Laboratoires Sérobiologiques sous la dénomination commerciale Filladyn LS 9397® ; le rétinol et ses esters dont le palmitate de rétinyl.

Agents favorisant la maturation de l'enveloppe cornée

On pourra utiliser dans les compositions de l'invention des agents qui interviennent sur la maturation de l'enveloppe cornée qui s'altère avec l'âge et induit une diminution de l'activité des transglutaminases. On peut citer par exemple l'urée et ses dérivés et en particulier l'Hydrovance® de National Starch et les autres actifs mentionnés dans la demande L'OREAL FR2877220.

Inhibiteurs de NO-synthases

L'agent ayant une action d'inhibiteur de NO synthase peut être choisi parmi les OPC (oligomères procyanidoliques) ; les extraits de végétal de l'espèce *Vitis vinifera* notamment commercialisés par la société Euromed sous la dénomination Leucocyanidines de raisins extra, ou encore par la société Indena sous la dénomination Leucoselect®, ou enfin par la société Hansen sous la dénomination Extrait de marc de raisin ; les extraits de végétal de l'espèce *Olea europaea* de préférence obtenus à partir de feuilles d'olivier et notamment commercialisés par la société VINYALS sous forme d'extrait sec, ou par la société Biologia & Technologia sous la dénomination commerciale Eurol® BT ; les extraits d'un végétal de l'espèce *Gingko biloba* de préférence un extrait aqueux sec de ce végétal vendu par la société Beaufour sous le nom commercial Ginkgo biloba extrait standard et leurs mélanges.

Agents stimulant le métabolisme énergétique des cellules

L'actif stimulant le métabolisme énergétique des cellules peut par exemple être choisi parmi la biotine, un extrait de *Saccharomyces cerevisiae* tel que le Phosphovital® de Sederma, le mélange de sels de sodium, de manganèse, de

zinc et de magnésium d'acide pyrrolidone carboxylique comme le Physiogenyl® de Solabia, un mélange de gluconate de zinc, de cuivre et de magnésium tel que le Sepitonic M3® de Seppic et leurs mélanges ; et un beta-glucan issu de *Saccharomyces cerevisiae,* tel que celui commercialisé par la société Mibelle AG Biochemistry.

L'invention porte également sur un procédé de traitement cosmétique de la peau destiné à diminuer ou prévenir les signes du vieillissement de la peau ou de ses phanères (cheveux, cils, ongles, ...), comprenant au moins une étape consistant à appliquer sur la peau au moins une composition telle que définie précédemment.

Le procédé selon l'invention comprend plus spécifiquement au moins une étape consistant à appliquer sur la peau de personnes présentant une peau présentant au moins l'un des signes de vieillissement cutané rappelés précédemment au moins une composition telle que définie précédemment.

Plus particulièrement, il comprend au moins une étape consistant à appliquer sur la peau de personnes présentant une peau ou une zone de peau vieillie, ridée, ou molle et/ou flasque ou sur des zones du corps présentant une perte d'élasticité et/ou de fermeté et/ou de tonicité au moins une composition telle que définie précédemment.

La composition selon l'invention peut être appliquée sur la partie de la peau ou des phanères à traiter, en particulier sur le visage, le corps, le cou, les mains, les cheveux ou le cuir chevelu, de préférence de façon quotidienne ou pluriquotidienne. L'application pourra être par exemple renouvelée tous les jours pendant une période variable selon les effets souhaités, généralement de 3 à 6 semaines, mais pourra être prolongée ou poursuivie en continu.

Selon une alternative, la composition selon l'invention peut être administrée par voie injectable en association ou non avec des produits de comblement. En effet, l'une des solutions retenues pour lutter contre les rides et/ou la perte de volume des tissus mous est l'utilisation de produits de comblement (ou "filler"). Ce comblement peut être réalisé par l'utilisation de produits non résorbables, tels que des gels de polyacrylamide ou des particules de polyméthylméthacrylate (PMMA). Néanmoins, ces composés peuvent entrainer des réactions d'intolérance du type inflammation ou hypersensibilité.

On privilégie l'utilisation de composants résorbables, tels que les protéines, les graisses, le collagène ou l'acide hyaluronique. Mais ces composés sont dégradés assez rapidement dans l'organisme, ce qui réduit leur efficacité. Pour y remédier il faut donc procéder à une réticulation plus ou moins poussée de ces composants. A ce jour, l'acide hyaluronique utilisé dans des formes pharmaceutiques ou des dispositifs médicaux se présente sous la forme d'un gel de hyaluronate de sodium. Le monosaccharide selon l'invention ou les compositions les contenant pourront également être appliqués par mésothérapie. La mésothérapie est une technique de traitement par injection intraépidermique et/ou intradermique et/ou sous-cutanée de produit(s) actif(s), comme par exemple des micro-nutriments, des vitamines, et/ou de l'acide hyaluronique. Les compositions sont administrées selon cette technique par injection sous forme de multiples gouttelettes de faible taille au niveau de l'épiderme, de la jonction dermo-épidermique et/ou du derme afin, notamment, de réaliser un nappage sous-cutané. La technique de mésothérapie est notamment décrite dans l'ouvrage « Traité de mésothérapie » de Jacques LE COZ, édition Masson, 2004. La mésothérapie faite sur le visage est également appelée mésolift, ou également sous le terme anglosaxon de « mesoglow ».

Ainsi, un autre objet de la présente invention peut être un dispositif, en particulier un dispositif médical, comprenant une quantité efficace d'au moins un monosaccharide tel que défini précédemment en association avec une quantité efficace d'au moins un C-glycoside ou un de ses dérivés. Ce dispositif peut être adapté à une injection intraépidermique et/ou intradermique et/ou sous-cutanée. L'association d'actifs telle que définie ci-dessus est mise en solution dans un milieu stérile. Ledit dispositif peut comprendre au moins un autre composé, comme au moins un produit résorbable ou non, tel que ceux mentionnés ci-dessus, éventuellement réticulé.

Ledit dispositif peut être par exemple une seringue avec une aiguille ou encore un dispositif injecteur sans aiguille, tel que ceux utilisés dans la technique de soin connue sous le nom de mésothérapie. Un kit comprenant un dispositif peut être également envisagé, ledit kit comprenant un dispositif, en particulier une seringue ou un dispositif injecteur, et au moins l'association d'actifs, monosaccharide(s) et C-glycoside ou dérivé(s), tel que défini ci-dessus. Ledit kit peut comprendre également une aiguille. Ledit dispositif peut se trouver prêt à l'emploi, c'est à dire pré-rempli, ou doit être rempli lors de l'utilisation. Dans ce dernier cas, une composition ou un autre dispositif (comme une ampoule) comprend ladite l'association d'actifs, monosaccharide(s) et C-glycoside ou dérivé(s), éventuellement en association avec au moins un autre composé actif, comme au moins un produit résorbable ou non, tel que les produits de comblement mentionnés ci-dessus, éventuellement réticulé.

L'injection de l'association selon l'invention peut être réalisée simultanément à, ou avant ou après, l'application sur la peau ou ses phanères d'une autre composition cosmétique ou pharmaceutique, de préférence dermatologique, comprenant, dans un support physiologiquement acceptable, au moins un autre actif, tel que cité ci-dessus.

Selon un autre aspect, l'invention concerne également un ensemble cosmétique comprenant : i) un récipient délimitant au moins un compartiment, ledit récipient étant fermé par un élément de fermeture ; et ii) une composition telle que décrite précédemment et disposée à l'intérieur dudit compartiment.

Le récipient peut être sous toute forme adéquate. Il peut être notamment sous forme d'un flacon, d'un tube, d'un pot, d'un étui, d'une boite, d'un sachet ou d'un boîtier. L'élément de fermeture peut être sous forme d'un bouchon amovible, d'un couvercle, d'un opercule, d'une bande déchirable, ou d'une capsule, notamment du type comportant un

corps fixé au récipient et une casquette articulée sur le corps. Il peut être également sous forme d'un élément assurant la fermeture sélective du récipient, notamment une pompe, une valve, ou un clapet.

Le récipient peut être associé à un applicateur. L'applicateur peut être sous forme d'un pinceau, tel que décrit par exemple dans le brevet FR 2 722 380. Le produit peut être contenu directement dans le récipient, ou indirectement. A titre d'exemple, le produit peut être disposé sur un support imprégné, notamment sous forme d'une lingette ou d'un tampon, et disposé (à l'unité ou plusieurs) dans une boîte ou dans un sachet. Un tel support incorporant le produit est décrit par exemple dans la demande WO 01/03538.

L'élément de fermeture peut être couplé au récipient par vissage.

Alternativement, le couplage entre l'élément de fermeture et le récipient se fait autrement que par vissage, notamment via un mécanisme à baïonnette, par encliquetage, serrage, soudage, collage, ou par attraction magnétique. Par " encliquetage " on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.

Le récipient peut être au moins pour partie réalisé en matériau thermoplastique. A titre d'exemples de matériaux thermoplastiques, on peut citer le polypropylène ou le polyéthylène.

Alternativement, le récipient est réalisé en matériau non thermoplastique, notamment en verre ou en métal (ou alliage).

Le récipient peut être à parois rigides ou à parois déformables, notamment sous forme d'un tube ou d'un flacon tube. Le récipient peut comprendre des moyens destinés à provoquer ou faciliter la distribution de la composition. A titre d'exemple, le récipient peut être à parois déformables de manière à provoquer la sortie de la composition en réponse à une surpression à l'intérieur du récipient, laquelle surpression est provoquée par écrasement élastique (ou non élastique) des parois du récipient.

Le contenu des brevets ou demandes de brevets cités précédemment sont incorporés par référence dans la présente demande.

Selon un mode particulier, l'invention concerne un ensemble cosmétique comprenant :

- une composition A contenant au moins un composé choisi parmi les C-glycosides, leurs dérivés et leurs mélanges,
- une composition B, conditionnée de manière séparée de la composition A, comprenant au moins un monosaccharide choisi parmi le mannose, le rhamnose et leur mélange.

L'invention concerne enfin un procédé de traitement cosmétique ou dermatologique comprenant au moins une étape d'administration, en particulier d'application topique, sur la peau et/ou ses phanères, de la composition A et au moins une étape d'administration, en particulier d'application topique sur la peau et/ou ses phanères, de la composition B.

L'administration de la composition A selon l'invention peut être réalisée simultanément à, ou avant ou après, l'administration de la composition B. Comme spécifié précédemment, l'administration de la composition A et de la composition B peut être réalisée par voie topique, orale ou par injection.

Selon une alternative, on administre en premier lieu la composition A et en second lieu la composition B. Selon une autre alternative, on administre en premier lieu la composition B et en second lieu la composition A.

Les compositions A et B peuvent être conditionnées séparément à l'intérieur de deux compartiments, formés soit par deux récipients distincts, soit à l'intérieur d'un dispositif unitaire. Par " dispositif unitaire " on entend un dispositif par lequel les deux compartiments sont solidaires l'un de l'autre. Un tel dispositif peut être obtenu par un procédé de moulage en une seule pièce des deux compartiments, notamment en un matériau thermoplastique. Il peut également résulter de toute forme d'assemblage, notamment par collage, soudage, ou autre encliquetage.

Selon un premier mode de réalisation, les deux récipients sont indépendants l'un de l'autre. De tels récipients peuvent se présenter sous diverses formes. Il peut s'agir notamment de tubes, de flacons ou de bidons.

L'un et/ou l'autre des récipients peuvent être surmontés d'une pompe à actionnement manuel surmontée d'un bouton poussoir pour l'actionnement de la pompe et la distribution de la composition via au moins un orifice de distribution.

Alternativement, l'un et/ou l'autre des récipients sont pressurisés, notamment au moyen d'un agent propulseur, en particulier un gaz propulseur. Dans ce cas, le (ou les) récipient(s) est (sont) équipé(s) d'une valve surmontée par un bouton poussoir équipée d'une buse ou de tout autre moyen de diffusion pour la distribution du produit.

Le propulseur peut être en mélange avec la composition à distribuer ou séparé, notamment via un piston apte à coulisser à l'intérieur du récipient, ou via les parois souples d'une poche à l'intérieur de laquelle est disposée la composition.

Les récipients peuvent être constitués de matériaux divers : plastique, verre, ou métal.

Alternativement encore, les deux compartiments sont formés de deux compartiments concentriques formés à l'intérieur d'un tube, et sont surmontés d'une pompe sans reprise d'air équipée d'un bouton poussoir à un ou deux orifices de distribution. A l'intérieur du tube est prévu un piston qui remonte en direction de la pompe au fur et à mesure que les compositions sont prélevées à l'intérieur des récipients. De tels modes de distribution sont utilisés notamment

pour la distribution de pâtes dentifrices.

**Légendes des Figures**

**Figure 1 :** Diagramme schématisant les résultats obtenus pour la prolifération des kératinocytes, en présence d'un témoin, en présence de différents marqueurs, en milieu carencé en facteurs de croissance, et avec ajout de différentes concentrations en L-Rhamnose reportées en abscisse. Les valeurs reportées en ordonnée correspondent aux pourcentages de cellules marquées mesurés par rapport au témoin.

**Figure 2 :** Diagramme schématisant les résultats obtenus pour la prolifération des kératinocytes, en présence d'un témoin, en présence de différents marqueurs, en milieu carencé en facteurs de croissance, et avec ajout de différentes concentrations en D-Mannose reportées en abscisse. Les valeurs reportées en ordonnée correspondent aux pourcentages de cellules marquées mesurés par rapport au témoin.

**Figure 3 :** Diagramme représentant le nombre de fibroblastes mesurés entre une peau reconstruite complète témoin non traité, à gauche, et une peau reconstruite complète traitée par 5 mM de rhamnose, à droite. Les fibroblastes sont comptés à différents stades du traitement. Ainsi, pour chaque type de peau la colonne de gauche correspond à la numérotation effectuée à 48 h et la colonne de droite correspond à la numérotation effectuée à 120 h de traitement.

**Figure 4 :** Photographies de coupe à congélation de peau reconstruite de 7μm d'épaisseur. Le niveau de fluorescence se matérialise par les taches blanches du cliché en noir et blanc, il est proportionnel à la quantité de procollagène de type I. A gauche figure la peau témoin et à droite la peau traitée par 1 mM de rhamnose.

L'invention est illustrée plus en détail dans les exemples suivants qui sont présentés à titre illustratif et non limitatif du domaine de l'invention.

**Exemples**

**Exemple 1 : Prolifération des kératinocytes**

**Protocole**

Les kératinocytes (lignée HaCat) sont cultivés dans deux conditions : milieu de culture défini complet (condition standard) et milieu de culture carencé en facteurs de croissance. Ce milieu carencé entraîne un retard maitrisé de la prolifération cellulaire. Dans ces conditions, il est alors possible de mesurer les effets de composés, capables de compenser la carence en facteurs de croissance du milieu de culture et donc de relancer la multiplication cellulaire et /ou de stimuler leur métabolisme.

La prolifération kératinocytaire est mesurée au moyen de trois marqueurs sur la même population cellulaire : le taux d'ADN qui est proportionnel au nombre de cellules (sonde Cyquant), le taux de lipides polaires constitutifs de membranes cellulaires (sonde Rouge Nil) et la respiration mitochondriale, qui réflète le métabolisme cellulaire général (sonde XTT).

**Résultats**

Les résultats sont donnés dans les figures 1 et 2.

Les deux monosaccharides Rhamnose et Mannose démontrent leur capacité à activer la prolifération des kératinocytes, lorsque ceux-ci sont cultivés en milieu appauvri en facteurs de croissance, condition de culture qui retarde significativement leur croissance cellulaire.

Cette activation de la prolifération cellulaire par les deux composés se manifeste par un nombre de cellules plus important par rapport au témoin non traité.

Ce nombre augmenté de cellules se matérialise par un taux d'ADN (Cyquant), un taux de lipides polaires (signal Rouge Nil) et une respiration mitochondriale (signal XTT) significativement augmentés lorsque les monosaccharides sont évalués à 1 mM. A 500 μM, les deux molécules présentent déjà une efficacité.

Les deux monosaccharides mannose et rhamnose exercent donc une influence sur la prolifération des kératinocytes. Ils activent la prolifération des kératinocytes cultivés en milieu appauvri en facteur de croissance, ce qui se manifeste par un nombre de cellules plus important par rapport au témoin non traité.

Le rhamnose et le mannose présentent donc une efficacité anti âge intéressante en venant booster le renouvellement épidermique et lutter contrer l'atrophie épidermique liée au vieillissement.

**Exemple 2 : Prolifération des fibroblastes**

**Protocole**

Le rhamnose a été étudié sur un modèle de peau reconstruite complète afin de mesurer son efficacité anti âge au niveau du compartiment dermique. Brièvement, le modèle de peau reconstruite utilisé est celui décrit par Bell et al, (Bell E. et al, The reconstitution of living skin, J Invest Dermatol, 1983, Jul ;81*) :* il comporte un équivalent dermique sur lequel est reconstruit un épiderme pluristratifié ; l'équivalent dermique est fabriqué à partir de collagène acido soluble, de milieu de culture contenant du sérum et de fibroblastes humains normaux adultes. Après 5 jours de rétraction, cet équivalent est ensemencé avec des kératinocytes puis cultivé durant 6 jours en immersion et 7 jours à émersion afin d'obtenir un épiderme pluristratifié et différencié présentant une couche cornée.

La peau reconstruite est traitée par du rhamnose à 5 mM pendant 2 jours et 5 jours dans le milieu de culture ; à l'issue du traitement, les peaux reconstruites sont incluses en Tissue Tek en vue de coupes à congélation de 7$\mu$M d'épaisseur au cryostat. Les coupes réalisées sont ensuite marquées à l'iodure de propidium pour marquer l'ADN des noyaux des fibroblastes en vue de leur numération. 3 coupes à congélation sont réalisées aléatoirement sur chaque peau reconstruite ; sur chaque coupe, 2 champs microscopiques (objectif X25) sont analysés en microscopie à fluorescence et photographiés. La numération des fibroblastes dermiques est donc réalisée pour chaque peau reconstruite sur 6 images au total représentant les 6 champs microscopiques considérés. Le nombre de fibroblastes dermiques est comparé entre la peau témoin et celle traitée par le rhamnose aux deux temps de la cinétique.

**Résultats**

Les résultats sont donnés à la figure 3.
Il a été constaté que le rhamnose induit la stimulation de la croissance des fibroblastes dermiques de la peau reconstruite dès 48 heures de traitement, stimulation confirmée à 120 h de traitement, avec entre 30 à 35% de cellules en plus (voir figure 3). A noter que cette stimulation s'accompagne d'une stimulation de la synthèse de procollagène 1 à 5 mM, ainsi qu'à 1 mM, ce qui peut également résulter du nombre accru des fibroblastes responsable de la sécrétion de cette protéine majeure de la matrice extracellulaire.

Ces deux effets complètent l'activité anti-âge du rhamnose déjà mesurée sur le compartiment épidermique, en venant stimuler la prolifération et le métabolisme du fibroblaste, cellule majeure du compartiment dermique.

**Exemple 3 : Synthèse de Procollagène 1**

Il a été procédé également sur d'autres séries de coupes à congélation à la détection classique par immunofluorescence indirecte du procollagène de type I au niveau du derme de la peau reconstruite (Anticorps anti procoll 1 *(MAB 1912 Millipore)* + conjugué couplé à FITC (*112-095-068 Jackson Immunoresearch)*). Afin de bien se repérer au sein de l'architecture cutanée lors de l'examen microscopique des coupes, les noyaux cellulaires des kératinocytes et des fibroblastes sont localisés grâce à leur marquage à l'iodure de propidium, comme décrit plus haut. 3 coupes à congélation sont réalisées aléatoirement sur chaque peau reconstruite et sur chaque coupe, 2 champs microscopiques (objectif X25) sont analysés en microscopie à fluorescence et photographiés. Les niveaux de fluorescence proportionnels à la quantité de procollagène de type I sont comparés entre la peau témoin et la peau traitée par le rhamnose.

Sur l'image 1, figure 4, correspondant à une coupe de la peau reconstruite témoin à 120h de culture, la présence du procollagène de type 1 synthétisé par les fibroblastes dermiques, se matérialise par la fluorescence verte située dans la partie inférieure de l'image. On devine sur la partie supérieure de l'image la partie basale de l'épiderme, tissu très cellulaire, visualisable par les nombreux noyaux des kératinocytes. On visualise également dans le derme ; tissu beaucoup moins cellulaire, la distribution aléatoire des fibroblastes au sein de la matrice extracellulaire dermique. Sur l'image 2, figure 4, correspondant par exemple, à une coupe de la peau reconstruite traitée par le Rhamnose à 1mM pendant 120 heures, on constate une nette augmentation de la fluorescence verte comparativement à celle observée pour la peau témoin (image 1) ainsi qu'une distribution du signal fluorescent matérialisant bien l'aspect fibrillaire du procollagène de type I néosynthétisé. Cette augmentation de la fluorescence générale indique que le traitement par le rhamnose a fortement stimulé la synthèse du procollagène de type I par les fibroblastes.

Ces résultats montrent bien la capacité du rhamnose à stimuler le métabolisme du fibroblaste, métabolisme qui au cours du vieillissement, se déséquilibre plus vers la dégradation de la matrice extracellulaire que vers son renouvellement.

Le rhamnose en stimulant à la fois le métabolisme et la croissance des fibroblastes dermiques démontre bien

son efficacité anti-âge sur le derme, efficacité complémentaire de celle mesurée vis-à-vis du compartiment épidermique.

**<u>Exemple 4 :</u> Association Rhamnose et C-Xyloside, mise en évidence de la complémentarité d'action du C-Xyloside et du Rhamnose sur la physiologie cutanée**

**Protocole**

Pour l'étude des effets de l'adjonction de l'association C-xyloside/rhamnose sur une peau reconstruite, on procède à :

- L'observation au microscope de coupe de peau avec un marquage immunohistochimique des protéines des collagène IV et VII ; et
- L'observation de l'épaisseur épidermique en histologie après coloration HES.

*1. Préparation de la peau reconstruite*

La peau reconstruite utilisée est réalisée selon le protocole décrit dans Asselineau et al. (Models in Dermato. Editions Loire and Maibach, 1987, Vol III, 1-7). Les modifications à ce protocole sont :

- l'utilisation de fibroblastes de derme humains normaux adultes à raison de $10^6$ cellules par derme équivalent ;
- l'ensemencement des kératinocytes se fait à raison de 50000 cellules par anneau de 1,5 cm de diamètre. Les kératinocytes utilisés proviennent d'un même donneur et sont en passage 1 lors de l'ensemencement des équivalents de derme ;
- la durée de la phase d'immersion est de 7 jours ;
- la durée de la phase d'émersion est de 7 jours.

*2. Addition de C-Xyloside et de Rhamnose*

La culture est ensuite montée en nacelle et traitée dans le milieu de culture par l'association C -xyloside/rhamnose pendant 5 jours avec changement du milieu tous les 2 jours.

*3.a. Analyse des collagènes IV et VII*

L'analyse des peaux reconstruites est réalisée en fin de traitement. Un échantillon témoin (absence de C-xyloside et rhamnose) est systématiquement réalisé et analysé en parallèle.

Chaque échantillon de peau est divisé en deux demi lunes, l'une pour l'analyse histologique, l'autre pour l'immunohistochimie sur coupes congelées. La première moitié des demi-lunes est congelée en azote liquide puis incluse en Tissue teck afin de constituer des blocs à congélation. Les collagènes de type IV et VII sont détectés par immunohistochimie sur des coupes congelées de 5μm. La technique classique d'immunofluorescence indirecte est réalisée avec un anticorps monoclonal anti-collagène VII (LH7.2, Chemicon International Inc., USA) et un conjugué couplé à la fluorescéine (FITC-conjugated Rabbit anti mouse immunoglobulins, DAKO, Denmark), et un anticorps monoclonal anti-collagène IV (MO785, DAKO Denmark) et un conjugué couplé à l'Alexa 488 (A11017 Invitrogen). Les noyaux cellulaires sont colorés à l'Iodure de propidium afin de bien repérer les différents compartiments de la peau reconstruite.

*3.b. Analyse de la prolifération épidermique.*

La seconde moitié des demi-lunes est incluse en paraffine en vue de coupes à paraffine de 5μm d'épaisseur ; les coupes sont ensuite colorées par la coloration HES.

Les coupes sont analysées en microscopie classique à fond clair. La prolifération épidermique est quantifiée par le dénombrement des assises de kératinocytes, par la mesure de l'épaisseur totale de l'épiderme malpighien, et ce, en comparant l'épiderme de la peau témoin, à celui de la peau traitée par l'association C-xyloside/rhamnose. L'ensemble de l'analyse est réalisée sur une station d'analyse d'image Zeiss + logiciel KS 300.

**Résultats**

On observe une augmentation de l'épaisseur de l'épiderme, un renforcement de la structure de la jonction dermo-épidermique, ainsi qu'une prolifération accrue des fibroblastes dermiques.

Ces effets, obtenus avec l'association du rhamnose et du C-xyloside, engendrent une régénération de la peau

en agissant sur ses trois compartiments principaux, démontrant ainsi une activité anti âge globale.

**Exemple 5 : exemple de réalisation d'une composition cosmétique selon l'invention**

| ***Crèmes régénération épidermiqe et dermique : émulsion huile dans eau*** | |
|---|---|
| Ammonium Polyacryldimethyltauramide (Hostacerin AMPS de Clariant) | 1.00% |
| Cyclohexasiloxane | 5.0% |
| Huile d'amande d'abricot | 7% |
| Isononyl isononanoate | 7% |
| Stearyl alcohol | 0.30% |
| Glyceryl stearate / PEG-100 stearate | 0.70% |
| Dimyristyl tartrate / cetearyl alcohol / C12-15 pareth-7 / PPG-25 laureth-25 | 0.50% |
| Gomme de Xanthan | 0,20 % |
| rhamnose | 5% |
| C-Xyloside | 3% |
| Conservateurs | 0.50% |
| Eau | qsp 100 |

**Exemple 6 : exemple de réalisation d'une composition cosmétique selon l'invention**

| ***Crèmes régénération épidermique : émulsion huile dans eau*** | |
|---|---|
| Ammonium Polyacryldimethyltauramide (Hostacerin AMPS de Clariant) | 1.00% |
| Cyclohexasiloxane | 5.0% |
| Glycerin | 1.70% |
| Stearyl alcohol | 0.30% |
| Huile d'amande d'abricot | 7% |
| Isononyl isononanoate | 7% |
| Dimyristyl tartrate / cetearyl alcohol / C12-15 pareth-7 / PPG-25 laureth-25 | 0.50% |
| Gomme de Xanthan | 0,20 % |
| mannose | 5% |
| C-Xyloside | 5% |
| Conservateurs | 0.50% |
| Eau | qsp 100 |

**Exemple 7 : exemple de réalisation d'une composition cosmétique selon l' invention**

**Crème de jour anti-âge pour le visage**

*Phase A* 1 :-

- Distéarate de sucrose commercialisé par la Société STEARINERIE DUBOIS 1,75%
- Stéarate de sorbitane oxyéthyléné à 4 moles d'oxyde d'éthylènecommercialisé par la Société ICI sous le nom "TWEEN 61" 1,15 %
- Acide stéarique 0,75 %

(suite)

| | |
|---|---|
| - Stearyl heptanoate | 4,00 % |
| - Vaseline codex | 1,50 % |
| - Huile d'avocat | 3,20 % |
| - Huile de jojoba | 3,00 % |
| - Huile de silicone volatile | 2,70 % |
| - Acétate de vitamine E | 1,00 % |
| - Glycérides de Vitamine F | 3,00 % |

*Phase A2 :*

| | |
|---|---|
| - Gomme de silicone commercialisée par DOW CORNING sous le nom "Q2-1403 Fluid" | 3,00 % |
| - Propylparaben | 0,2 % |
| - Parfum | 0,3 % |

*Phase B :*

| | | |
|---|---|---|
| - Glycérine | | 3,00 % |
| - Hydroxyproline | | 1,00 % |
| - D-panthenol | | 1,00 % |
| - Triéthanolamine | | 0,35 % |
| - Rhamnose | | 3,00% |
| - C-Xyloside | | 10,00% |
| - Méthylparaben | | 0,3 % |
| - Eau déminéralisée | q.s.p. | 100 % |

*Phase C :*

- Ammonium Polyacryldimethyltauramide (Hostacerin AMPS de Clariant) 1%

**Revendications**

**1.** Composition cosmétique comprenant, dans un milieu physiologiquement acceptable, l'association d'au moins un monosaccharide choisi parmi le mannose, le rhamnose et leur mélange, et d'au moins un composé additionnel choisi parmi les C-glycosides, leurs dérivés et leurs mélanges, dans laquelle ledit monosaccharide est sous forme de monomère, et dans laquelle le C-glycoside répond à la formule (I) suivante :

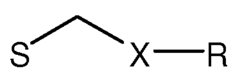

(I)

dans laquelle,

. R représente :

- un radical alkyle linéaire, saturé en C1 à C20, en particulier en C1 à C10, ou insaturé en C2 à C20, en particulier en C2 à C10, ou un radical alkyle ramifié ou cyclique, saturé ou insaturé, en C3 à C20, en particulier en C3 à C10 ;
- un radical hydrofluoro- ou perfluoro-alkyle, linéaire saturé en C1 à C20, en particulier en C1 à C10, ou insaturé en C2 à C20, en particulier en C2 à C10, ou ramifié ou cyclique, saturé ou insaturé, en C3 à C20, en particulier en C3 à C10 ; la chaîne hydrocarbonée constituant lesdits radicaux pouvant, le cas échéant,

être interrompue par 1, 2, 3 ou plus d'hétéroatomes choisis parmi :
- un oxygène,
- un soufre,
- un azote, et
- un silicium,

et pouvant être éventuellement substituée par au moins un radical choisi parmi :

- -OR4,
- -SR4,
- -NR4R5,
- -COOR4,
- -CONHR4,
- -CN,
- un atome d'halogène,
- un radical hydrofluoro- ou perfluoro-alkyle, en C1 à C6, et/ou
- un radical cycloalkyle en C3 à C8,

avec R4 et R5 pouvant représenter, indépendamment l'un de l'autre, un atome d'hydrogène, ou un radical alkyle, perfluoroalkyle ou hydrofluoroalkyle linéaire, saturé en C1 à C30, notamment en C1 à C12, ou insaturé en C2 à C30, notamment en C2 à C12, ou ramifié ou cyclique, saturé ou insaturé, en C3 à C30, notamment en C3 à C12 ; ou un radical aryle en C6 à C10,

. X représente un radical choisi parmi les groupements :

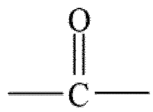

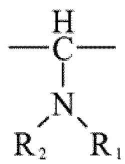

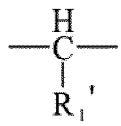

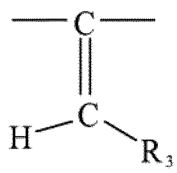

avec R1, R2 et R3 représentant, indépendamment l'un de l'autre, un atome d'hydrogène, ou un radical R, avec R tel que défini précédemment, et R'1 représente un atome d'hydrogène, un groupe -OH ou un radical R tel que défini précédemment, R1 pouvant désigner également un radical aryle en C6 à C10 ;
. S représente un monosaccharide ou un polysaccharide comportant jusqu'à 20 unités sucre, en particulier jusqu'à 6 unités sucre, sous forme pyranose et/ou furanose et de série L et/ou D, ledit mono- ou poly-saccharide

pouvant être substitué par un groupement hydroxyle obligatoirement libre, et éventuellement une ou plusieurs fonction(s) amine(s) éventuellement protégée(s), et
. la liaison S-CH2-X représente une liaison de nature C-anomérique, qui peut être [alpha] ou [beta],

ainsi que leurs sels cosmétiquement acceptables, leurs solvates tels que les hydrates et leurs isomères.

**2.** Composition selon la revendication 1, dans laquelle X représente un groupement choisi parmi -CO-, -CH(OH)-, -CH(NH2)-, et préférentiellement un groupement - CH(OH)-.

**3.** Composition selon la revendication 1 ou 2, dans laquelle S représente un monosaccharide choisi parmi le D-glucose, le D-xylose, le L-fucose, le D-galactose, le D-maltose et notamment le D-xylose.

**4.** Composition selon l'une quelconque des revendications précédentes, dans laquelle R désigne un radical linéaire en C1-C4, notamment C1-C3, éventuellement substitué par -OH, -COOH ou -COOR"2, R"2 étant un radical alkyle saturé en C1-C4, notamment éthyle.

**5.** Composition selon l'une quelconque des revendications précédentes, dans laquelle les dérivés C-glycosides sont choisis parmi le C-[beta]-D-xylopyranoside-n-propane-2-one, le C-[alpha]- D-xylopyranoside-n-propane-2-one, le C-[beta]-D-xylopyranoside-2-hydroxy-propane, le C-[alpha]- D-xylopyranoside-2-hydroxy-propane, la 1-(C-[beta]-D-fucopyranoside)-propane-2-one, la 1-(C-[alpha]-D-fucopyranoside)-propane-2-one, la 1-(C-[beta]-L-fucopyranoside)-propane-2-one, la 1-(C-[alpha]-L-fucopyranoside)-propane-2-one, le 1-(C-[beta]-D-fucopyranoside)-2-hydroxy-propane, le 1-(C-[alpha]-D-focopyranoside)-2-hydroxy-propane, le 1-(C-[beta]-L-fucopyranoside)-2-hydroxy-propane, le 1-(C-[alpha]-L-fucopyranoside) -2-hydroxy-propane, le 1-(C-[beta]-D-glucopyranosyl)-2-hydroxyl-propane, le 1-(C-[alpha]-D-glucopyranosyl)-2-hydroxyl-propane, le 1-(C-[beta]-D-galactopyranosyl)-2-hydroxyl-propane, le 1-(C-[alpha]-D-galactopyranosyl)-2-hydroxyl-propane, la 1-(C-[beta]-D-fucofuranosyl)-propane-2-one, la 1-(C-[alpha]-D-fucofuranosyl)-propane-2-one, la 1-(C-[beta]-L-fucofuranosyl)-propane-2-one, la 1-(C-[alpha]-L-fucofuranosyl)-propane-2-one, le C-[beta]-D-maltopyranoside-n-propane-2-one, le C-[alpha]-D-maltopyranoside-n-propane-2-one, le C-[beta]-D-maltopyranoside-2-hydroxy-propane, le C-[alpha]-D-maltopyranoside-2-hydroxy-propane, leurs isomères et leurs mélanges.

**6.** Composition selon l'une quelconque des revendications précédentes, dans laquelle les dérivés C-glycosides sont choisis parmi le C-[beta]-D-xylopyranoside-2-hydroxy-propane et le C-[alpha]- D-xylopyranoside-2-hydroxy-propane, de préférence le C-[beta]-D-xylopyranoside-2-hydroxy-propane.

**7.** Composition selon l'une quelconque des revendications précédentes, dans laquelle le monosaccharide est le rhamnose.

**8.** Composition selon l'une quelconque des revendications 1 à 6, dans laquelle le monosaccharide est le mannose.

**9.** Composition selon l'une quelconque des revendications précédentes, dans laquelle la quantité dudit (desdits) monosaccharide(s) est comprise entre 0,001 % et 30 % en poids par rapport au poids total de la composition, et en particulier entre 0,1 % et 10 % en poids, et plus particulièrement entre 0,5 % et 6 % en poids par rapport au poids total de la composition.

**10.** Composition selon l'une quelconque des revendications précédentes, dans laquelle la quantité dudit (desdits) composé(s) additionnel(s) est comprise entre 0,001 % et 30 % en poids par rapport au poids total de la composition, et en particulier entre 0,1 % et 10 % en poids, et plus particulièrement entre 0,5 % et 6 % en poids par rapport au poids total de la composition.

**11.** Utilisation cosmétique d'une composition selon l'une quelconque des revendications précédentes, pour diminuer et/ou prévenir les signes du vieillissement de la peau ou de ses phanères.

**12.** Utilisation cosmétique d'une composition selon l'une quelconque des revendications 1-10, pour améliorer la densité de la peau, sa fermeté et/ou pour traiter, de manière préventive ou curative, l'amincissement du derme, la dégradation des fibres de collagène, la peau molle et/ou la peau amincie.

**13.** Utilisation cosmétique d'une composition selon l'une quelconque des revendications 1-10, pour augmenter la synthèse des laminines, de préférence la laminine V, et la synthèse des collagènes, de préférence choisis parmi le

pro-collagène I, le collagène IV et le collagène VII.

**14.** Dispositif comprenant au moins, l'association d'au moins un monosaccharide choisi parmi le mannose, le rhamnose et leur mélange, et d'au moins un composé additionnel choisi parmi les C-glycosides, leurs dérivés et leurs mélanges, le dispositif étant adapté à une injection intraépidermique et/ou intradermique et/ou sous-cutanée, dans lequel ledit monosaccharide est sous forme de monomère, et dans lequel le C-glycoside répond à la formule (I) suivante :

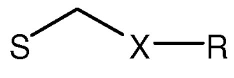

(I)

dans laquelle,

. R représente :

- un radical alkyle linéaire, saturé en C1 à C20, en particulier en C1 à C10, ou insaturé en C2 à C20, en particulier en C2 à C10, ou un radical alkyle ramifié ou cyclique, saturé ou insaturé, en C3 à C20, en particulier en C3 à C10 ;
- un radical hydrofluoro- ou perfluoro-alkyle, linéaire saturé en C1 à C20, en particulier en C1 à C10, ou insaturé en C2 à C20, en particulier en C2 à C10, ou ramifié ou cyclique, saturé ou insaturé, en C3 à C20, en particulier en C3 à C10 ; la chaîne hydrocarbonée constituant lesdits radicaux pouvant, le cas échéant, être interrompue par 1, 2, 3 ou plus d'hétéroatomes choisis parmi :
- un oxygène,
- un soufre,
- un azote, et
- un silicium,

et pouvant être éventuellement substituée par au moins un radical choisi parmi :

- -OR4,
- -SR4,
- -NR4R5,
- -COOR4,
- -CONHR4,
- -CN,
- un atome d'halogène,
- un radical hydrofluoro- ou perfluoro-alkyle, en C1 à C6, et/ou
- un radical cycloalkyle en C3 à C8,

avec R4 et R5 pouvant représenter, indépendamment l'un de l'autre, un atome d'hydrogène, ou un radical alkyle, perfluoroalkyle ou hydrofluoroalkyle linéaire, saturé en C1 à C30, notamment en C1 à C12, ou insaturé en C2 à C30, notamment en C2 à C12, ou ramifié ou cyclique, saturé ou insaturé, en C3 à C30, notamment en C3 à C12 ; ou un radical aryle en C6 à C10,

. X représente un radical choisi parmi les groupements :

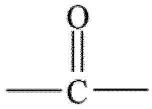

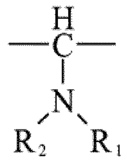

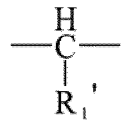

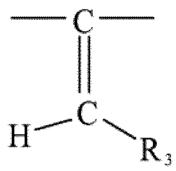

avec R1, R2 et R3 représentant, indépendamment l’un de l’autre, un atome d’hydrogène, ou un radical R, avec R tel que défini précédemment, et R’1 représente un atome d’hydrogène, un groupe -OH ou un radical R tel que défini précédemment, R1 pouvant désigner également un radical aryle en C6 à C10 ;
. S représente un monosaccharide ou un polysaccharide comportant jusqu’à 20 unités sucre, en particulier jusqu’à 6 unités sucre, sous forme pyranose et/ou furanose et de série L et/ou D, ledit mono- ou poly-saccharide pouvant être substitué par un groupement hydroxyle obligatoirement libre, et éventuellement une ou plusieurs fonction(s) amine(s) éventuellement protégée(s), et
. la liaison S-CH2-X représente une liaison de nature C-anomérique, qui peut être [alpha] ou [beta],

ainsi que leurs sels cosmétiquement acceptables, leurs solvates tels que les hydrates et leurs isomères.

**Patentansprüche**

**1.** Kosmetische Zusammensetzung, umfassend in einem physiologisch verträglichen Medium die Assoziation wenigstens eines Monosaccharids, das aus Mannose, Rhamnose und ihrem Gemisch ausgewählt ist, und wenigstens einer weiteren Verbindung, die aus C-Glycosiden, ihren Derivaten und ihren Gemischen ausgewählt ist, wobei besagtes Monosaccharid als Monomer vorliegt und wobei das C-Glycosid der folgenden Formel (I) entspricht:

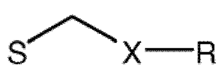

(I)

in der

- R steht für:
- einen gesättigten linearen C1-C20-Alkylrest, insbesondere C1-C10, oder einen ungesättigten linearen C2-C20-Alkylrest, insbesondere C2-C10, oder einen gesättigten oder ungesättigten, verzweigten oder cyclischen C3-C20-Alkylrest, insbesondere C3-C10;
- einen gesättigten linearen C1-C20-Hydrofluor- oder Perfluoralkylrest, insbesondere C1-C10, oder einen ungesättigten linearen C2-C20-Hydrofluor- oder Perfluoralkylrest, insbesondere C2-C10, oder einen gesättigten oder ungesättigten, verzweigten oder cyclischen C3-C20-Hydrofluor- oder Perfluoralkylrest, insbesondere C3-C10;

wobei die Kohlenwasserstoffkette, die besagte Reste bildet, gegebenenfalls durch 1, 2, 3 oder mehr Heteroatome

unterbrochen sein kann, die ausgewählt sind aus:

- Sauerstoff,
- Schwefel,
- Stickstoff, und
- Silicium,

und eventuell mit wenigstens einem Rest substituiert sein kann, der ausgewählt ist aus:

- -OR4,
- -SR4,
- -NR4R5,
- -COOR4,
- -CONHR4,
- -CN,
- einem Halogenatom,
- einem C1-C6-Hydrofluor- oder Perfluoralkylrest, und/oder
- einem C3-C8-Cycloalkylrest,

wobei R4 und R5 unabhängig voneinander für ein Wasserstoffatom oder einen gesättigten linearen C1-C30-Alkyl-, Perfluoralkyl- oder Hydrofluoralkylrest, insbesondere C1-C12, oder einen ungesättigten linearen C2-C30-Alkyl-, Perfluoralkyl- oder Hydrofluoralkylrest, insbesondere C2-C12, oder einen gesättigten oder ungesättigten, verzweigten oder cyclischen C3-C30-Alkyl-, Perfluoralkyl- oder Hydrofluoralkylrest, insbesondere C3-C12; oder einen C6-C10-Arylrest stehen können,

- X für einen Rest steht, der ausgewählt ist aus den Gruppen:

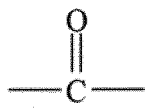

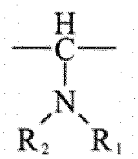

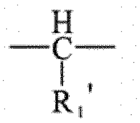

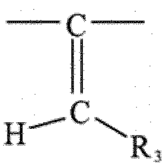

wobei R1, R2 und R3 unabhängig voneinander für ein Wasserstoffatom oder einen Rest R stehen, wobei R wie zuvor definiert ist und R'1 für ein Wasserstoffatom, eine OH-Gruppe oder einen wie zuvor definierten Rest R steht, wobei R1 ebenfalls einen C6-C10-Arylrest bezeichnen kann;
- S für ein Monosaccharid oder ein Polysaccharid steht, umfassend bis zu 20 Zuckereinheiten, insbesondere bis zu 6 Zuckereinheiten, in Pyranose- und/oder Furanose-Form und als D-und/oder L-Reihe, wobei besagtes

Mono- oder Polysaccharid mit einer obligat freien Hydroxylgruppe und eventuell mit einer oder mehreren eventuell geschützten Aminfunktionen substituiert sein kann, und
- die S-CH2-X-Bindung für eine C-anomere Bindung steht, die [alpha] oder [beta] sein kann, sowie ihre kosmetisch verträglichen Salze, ihre Solvate wie beispielsweise die Hydrate oder ihre Isomere.

**2.** Zusammensetzung gemäß Anspruch 1, wobei X für eine Gruppe steht, die aus einer -CO-, - CH(OH)-, -CH(NH2)- und vorzugsweise einer -CH(OH)-Gruppe ausgewählt ist.

**3.** Zusammensetzung gemäß Anspruch 1 oder 2, wobei S für ein Monosaccharid steht, das aus D-Glucose, D-Xylose, L-Fucose, D-Galactose, D-Maltose und insbesondere D-Xylose ausgewählt ist.

**4.** Zusammensetzung gemäß einem der vorhergehenden Ansprüche, wobei R einen linearen C1-C4-Rest, insbesondere einen C1-C3-Rest, bezeichnet, der eventuell mit -OH, -COOH oder - COOR"2 substituiert ist, wobei R"2 ein gesättigter C1-C4-Rest, insbesondere ein Ethylrest, ist.

**5.** Zusammensetzung gemäß einem der vorhergehenden Ansprüche, wobei die C-Glycosid-Derivate aus C-[beta]-D-Xylopyranosid-n-propan-2-on, C-[alpha]-D-Xylopyranosid-n-propan-2-on, C-[beta]-D-Xylopyranosid-2-hydroxypropan, C-[alpha]-D-Xylopyranosid-2-hydroxypropan, 1-(C-[beta]-D-Fucopyranosid)-propan-2-on, 1-(C-[alpha]-D-Fucopyranosid)-propan-2-on, 1-(C-[beta]-L-Fucopyranosid)-propan-2-on, 1-(C-[alpha]-L-Fucopyranosid)-propan-2-on, 1-(C-[beta]-D-Fucopyranosid)-2-hydroxy-propan, 1-(C-[alpha]-D-Fucopyranosid)-2-hydroxy-propan, 1-(C-[beta]-L-Fucanopyranosid)-2-hydroxy-propan, 1-(C-[alpha]-L-Fucopyranosid)-2-hydroxy-propan, 1-(C-[beta]-D-Glucopyranosyl)-2-hydroxyl-propan, 1-(C-[alpha]-D-Glucopyranosyl)-2-hydroxyl-propan, 1-(C-[beta]-D-Galactopyranosyl)-2-hydroxyl-propan, 1-(C-[alpha]-D-Galactopyranosyl)-2-hydroxyl-propan, 1-(C-[beta]-D-Fucofuranosyl)-propan-2-on, 1-(C-[alpha]-D-Fucofuranosyl)-propan-2-on, 1-(C-[beta]-L-Fucofuranosyl)-propan-2-on, 1-(C-[alpha]-L-Fucofuranosyl)-propan-2-on, C-[beta]-D-Maltopyranosid-n-propan-2-on, C-[alpha]-D-Maltopyranosid-n-propan-2-on, C-[beta]-D-Maltopyranosid-2-hydroxy-propan, C-[alpha]-D-Maltopyranosid-2-hydroxy-propan, ihren Isomeren und ihren Gemischen ausgewählt sind.

**6.** Zusammensetzung gemäß einem der vorhergehenden Ansprüche, wobei die C-Glycosid-Derivate aus C-[beta]-D-Xylopyranosid-2-hydroxy-propan und C-[alpha]-D-Xylopyranosid-2-hydroxy-propan, vorzugsweise C-[beta]-D-Xylopyranosid-2-hydroxy-propan, ausgewählt sind.

**7.** Zusammensetzung gemäß einem der vorhergehenden Ansprüche, wobei das Monosaccharid Rhamnose ist.

**8.** Zusammensetzung gemäß einem der Ansprüche 1 bis 6, wobei das Monosaccharid Mannose ist.

**9.** Zusammensetzung gemäß einem der vorhergehenden Ansprüche, wobei die Menge an besagtem oder besagten Monosacchariden zwischen 0,001 Gew.-% und 30 Gew.-% bezogen auf das Gesamtgewicht der Zusammensetzung und insbesondere zwischen 0,1 Gew.-% und 10 Gew.-% und ganz besonders zwischen 0,5 Gew.-% und 6 Gew.-% bezogen auf das Gesamtgewicht der Zusammensetzung beträgt.

**10.** Zusammensetzung gemäß einem der vorhergehenden Ansprüche, wobei die Menge an besagter oder besagten weiteren Verbindungen zwischen 0,001 Gew.-% und 30 Gew.-% bezogen auf das Gesamtgewicht der Zusammensetzung und insbesondere zwischen 0,1 Gew.-% und 10 Gew.-% und ganz besonders zwischen 0,5 Gew.-% und 6 Gew.-% bezogen auf das Gesamtgewicht der Zusammensetzung beträgt.

**11.** Kosmetische Verwendung einer Zusammensetzung gemäß einem der vorhergehenden Ansprüche zur Verringerung und/oder Prävention von Anzeichen der Alterung der Haut oder ihren Hautanhangsgebilden.

**12.** Kosmetische Verwendung einer Zusammensetzung gemäß einem der Ansprüche 1 bis 10 zur Verbesserung der Dichte, der Festigkeit der Haut und/oder zur präventiven oder kurativen Behandlung des Dünnerwerdens der Dermis, des Abbaus von Kollagenfasern, der schlaffen Haut und/oder der dünner gewordenen Haut.

**13.** Kosmetische Verwendung einer Zusammensetzung gemäß einem der Ansprüche 1 bis 10, zur Steigerung der Synthese von Lamininen, vorzugsweise Laminin V, und der Synthese von Kollagenen, die vorzugsweise aus Prokollagen I, Kollagen IV und Kollagen VII ausgewählt sind.

**14.** Vorrichtung, umfassend wenigstens die Assoziation wenigstens eines Monosaccharids, das aus Mannose, Rhamnose und ihrem Gemisch ausgewählt ist, und wenigstens einer weiteren Verbindung, die aus C-Glycosiden, ihren Derivaten und ihren Gemischen ausgewählt ist, wobei die Vorrichtung für eine intraepidermale und/oder intradermale und/oder subkutane Injektion angepasst ist, wobei besagtes Monosaccharid als Monomer vorliegt und wobei das C-Glycosid der folgenden Formel (I) entspricht:

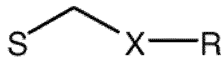

(I)

in der

- R steht für:
- einen gesättigten linearen C1-C20-Alkylrest, insbesondere C1-C10, oder einen ungesättigten linearen C2-C20-Alkylrest, insbesondere C2-C10, oder einen gesättigten oder ungesättigten, verzweigten oder cyclischen C3-C20-Alkylrest, insbesondere C3-C10;
- einen gesättigten linearen C1-C20-Hydrofluor- oder Perfluoralkylrest, insbesondere C1-C10, oder einen ungesättigten linearen C2-C20-Hydrofluor- oder Perfluoralkylrest, insbesondere C2-C10, oder einen gesättigten oder ungesättigten, verzweigten oder cyclischen C3-C20-Hydrofluor- oder Perfluoralkylrest, insbesondere C3-C10;

wobei die Kohlenwasserstoffkette, die besagte Reste bildet, gegebenenfalls durch 1, 2, 3 oder mehr Heteroatome unterbrochen sein kann, die ausgewählt sind aus:

- Sauerstoff,
- Schwefel,
- Stickstoff, und
- Silicium,

und eventuell mit wenigstens einem Rest substituiert sein kann, der ausgewählt ist aus:

- -OR4,
- -SR4,
- -NR4R5,
- -COOR4,
- -CONHR4,
- -CN,
- einem Halogenatom,
- einem C1-C6-Hydrofluor- oder Perfluoralkylrest, und/oder
- einem C3-C8-Cycloalkylrest,

wobei R4 und R5 unabhängig voneinander für ein Wasserstoffatom oder einen gesättigten linearen C1-C30-Alkyl-, Perfluoralkyl- oder Hydrofluoralkylrest, insbesondere C1-C12, oder einen ungesättigten linearen C2-C30-Alkyl-, Perfluoralkyl- oder Hydrofluoralkylrest, insbesondere C2-C12, oder einen gesättigten oder ungesättigten, verzweigten oder cyclischen C3-C30-Alkyl-, Perfluoralkyl- oder Hydrofluoralkylrest, insbesondere C3-C12; oder einen C6-C10-Arylrest stehen können,

- X für einen Rest steht, der ausgewählt ist aus den Gruppen:

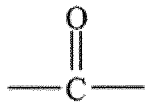

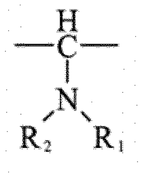

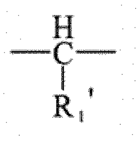

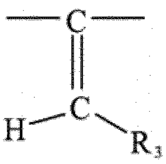

wobei R1, R2 und R3 unabhängig voneinander für ein Wasserstoffatom oder einen Rest R stehen, wobei R wie zuvor definiert ist und R'1 für ein Wasserstoffatom, eine OH-Gruppe oder einen wie zuvor definierten Rest R steht, wobei R1 ebenfalls einen C6-C10-Arylrest bezeichnen kann;

- S für ein Monosaccharid oder ein Polysaccharid steht, umfassend bis zu 20 Zuckereinheiten, insbesondere bis zu 6 Zuckereinheiten, in Pyranose- und/oder Furanose-Form und als D-und/oder L-Reihe, wobei besagtes Mono- oder Polysaccharid mit einer obligat freien Hydroxylgruppe und eventuell mit einer oder mehreren eventuell geschützten Aminfunktionen substituiert sein kann, und
- die S-CH2-X-Bindung für eine C-anomere Bindung steht, die [alpha] oder [beta] sein kann,

sowie ihre kosmetisch verträglichen Salze, ihre Solvate wie beispielsweise die Hydrate oder ihre Isomere.

## Claims

**1.** A cosmetic composition comprising, in a physiologically acceptable medium, a combination of at least one monosaccharide chosen from mannose, rhamnose and a mixture thereof, and at least one additional compound chosen from C-glycosides, derivatives and mixtures thereof, wherein said monosaccharide is as a monomer, and wherein the C-glycoside has the following formula (I):

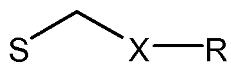

(I)

in which:

• R represents:

- a saturated $C_1$-$C_{20}$, preferably $C_1$-$C_{10}$, or unsaturated $C_2$-$C_{20}$, preferably $C_2$-$C_{10}$, linear alkyl radical, or a saturated or unsaturated, branched or cyclic $C_3$-$C_{20}$, preferably $C_3$-$C_{10}$, alkyl radical;
- a saturated $C_1$-$C_{20}$, preferably $C_1$-$C_{10}$, or unsaturated $C_2$-$C_{20}$, preferably $C_2$-$C_{10}$, linear, or saturated or unsaturated, branched or cyclic $C_3$-$C_{20}$, preferably $C_3$-$C_{10}$, hydrofluoroalkyl or perfluoroalkyl radical; the hydrocarbon-based chain constituting said radicals optionally being interrupted with 1, 2, 3 or more heteroatoms chosen from:
- oxygen,
- sulfur,

- nitrogen, and
- silicon,

and optionally being substituted with at least one radical chosen from:

- $-OR_4$,
- $-SR_4$,
- $-NR_4R_5$,
- $-COOR_4$,
- $-CONHR_4$,
- -CN,
- a halogen atom,
- a $C_1$-$C_6$ hydrofluoroalkyl or perfluoroalkyl radical, and/or
- a $C_3$-$C_8$ cycloalkyl radical,

with $R_4$ and $R_5$ representing, independently of each other, a hydrogen atom or a saturated $C_1$-$C_{30}$, in particular $C_1$-$C_{12}$, or unsaturated $C_2$-$C_{30}$, in particular $C_2$-$C_{12}$, linear, or a saturated or unsaturated, branched or cyclic $C_3$-$C_{30}$, in particular $C_3$-$C_{12}$, alkyl, perfluoroalkyl or hydrofluoroalkyl radical; or a $C_6$-$C_{10}$ aryl radical,

• X represents a radical chosen from:

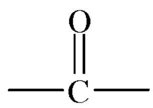

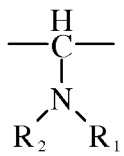

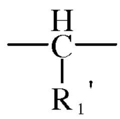

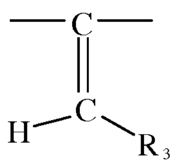

with $R_1$, $R_2$ and $R_3$ representing, independently of each other, a hydrogen atom or a radical R, with R as defined above, and $R'_1$ represents a hydrogen atom, an -OH group or a radical R as defined above, $R_1$ optionally also denoting a $C_6$-$C_{10}$ aryl radical;

• S represents a monosaccharide or a polysaccharide comprising up to 20 sugar units and in particular up to 6 sugar units, in pyranose and/or furanose form and of L and/or D series, said mono- or polysaccharide possibly being substituted with a mandatorily free hydroxyl group, and optionally one or more optionally protected amine function(s), and

• the bond $S-CH_2-X$ represents a bond of C-anomeric nature, which may be $\alpha$ or $\beta$, and the cosmetically acceptable salts, solvates such as hydrates, and isomers thereof.

**2.** The composition according to claim 1, in which X represents a group chosen from -CO-, -CH(OH)- and -CH(NH2), and preferably -CH(OH)-.

**3.** The composition according to claim 1 or 2, in which S represents a monosaccharide chosen from D-glucose, D-xylose, L-fucose, D-galactose and D-maltose, and in particular D-xylose.

**4.** The composition according to anyone of the preceding claims, in which R denotes a linear C1-C4 radical, in particular C1-C3, optionally substituted with -OH, -COOH or -COOR"2, R"2 being a C1-C4 saturated alkyl radical, in particular ethyl.

**5.** The composition according to anyone of the preceding claims, wherein the C-glycoside derivatives are chosen from C-[β]-D-xylopyranoside-n-propane-2-one, C-[a]-D-xylopyranoside-n-propane-2-one, C-[β]-D-xylopyranoside-2-hydroxypropane, C-[α]-D-xylopyranoside-2-hydroxypropane, 1-(C-[β]-D-fucopyranoside)propane-2-one, 1-(C-[a]-D-fucopyranoside)propane-2-one, 1-(C-[β]-L-fucopyranoside)propane-2-one, 1-(C-[α]-L-fucopyranoside)propane-2-one, 1-(C-[β]-D-fucopyranoside)-2-hydroxypropane, 1-(C-[α]-D-focopyranoside)-2-hydroxypropane, 1-(C-[β]-L-fucopyranoside)-2-hydroxypropane, 1-(C-[α]-L-fucopyranoside)-2-hydroxypropane, 1-(C-[β]-D-glucopyranosyl)-2-hydroxylpropane, 1-(C-[α]-D-glucopyranosyl)-2-hydroxylpropane, 1-(C-[β]-D-galactopyranosyl)-2-hydroxylpropane, 1-(C-[α]-D-galactopyranosyl)-2-hydroxylpropane, 1-(C-[β]-D-fucofuranosyl)propane-2-one, 1-(C-[α]-D-fucofuranosyl)propane-2~one, 1-(C-[β]-L-fucofuranosyl)propane-2-one, 1-(C-[α]-L-fucofuranosyl)propane-2-one, C-[β]-D-maltopyranoside-n-propane-2-one, C-[α]-D-maltopyranoside-n-propane-2-one, C-[β]-D-maltopyranoside-2-hydroxypropane, C-[α]-D-maltopyranoside-2-hydroxypropane, isomers thereof and mixtures thereof.

**6.** The composition according anyone of the preceding claims, in which the C-glycoside derivatives are chosen from C-[β]-D-xylopyranoside-2-hydroxypropane and C-[α]-D-xylopyranoside-2-hydroxypropane, preferably C-[β]-D-xylopyranoside-2-hydroxypropane.

**7.** The composition according anyone of the preceding claims, wherein the monosaccharide is rhamnose.

**8.** The composition according anyone of the preceding claims, wherein the monosaccharide is mannose.

**9.** The composition according to any one of the preceding claims, in which the amount of the said monosaccharide(s) is between 0.001% and 30% by weight relative to the total weight of the composition, in particular between 0.1 % and 10% by weight and more particularly between 0.5% and 6% by weight relative to the total weight of the composition.

**10.** The composition according to any one of the preceding claims, in which the amount of the said additional compound(s) is between 0.001% and 30% by weight relative to the total weight of the composition, in particular between 0.1 % and 10% by weight and more particularly between 0.5% and 6% by weight relative to the total weight of the composition.

**11.** A cosmetic use of a composition according to any one of the preceding claims, for reducing and/or preventing the signs of ageing of the skin or its integuments.

**12.** A cosmetic use of a composition according to any one of Claims 1 to 10, for improving the density and firmness of the skin and/or for preventively or curatively treating thinning of the dermis, degradation of collagen fibres, flaccid skin and/or thinned skin.

**13.** A cosmetic use of a composition according to any one of Claims 1 to 10, for increasing the synthesis of laminins, preferably laminin V, and the synthesis of collagens, preferably chosen from procollagen I, collagen IV and collagen VII.

**14.** A device comprising at least a combination of at least one monosaccharide chosen from mannose, rhamnose and a mixture thereof, and at least one additional compound chosen from C-glycosides, derivatives thereof and mixtures thereof, the device being suitable for intraepidermal and/or intradermal and/or subcutaneous injection, wherein the C-glycoside has the following formula (I):.

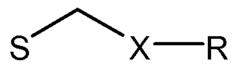

(I)

in which:

- R represents:

  - a saturated $C_1$-$C_{20}$, preferably $C_1$-$C_{10}$, or unsaturated $C_2$-$C_{20}$, preferably $C_2$-$C_{10}$, linear alkyl radical, or a saturated or unsaturated, branched or cyclic $C_3$-$C_{20}$, preferably $C_3$-$C_{10}$, alkyl radical;
  - a saturated $C_1$-$C_{20}$, preferably $C_1$-$C_{10}$, or unsaturated $C_2$-$C_{20}$, preferably $C_2$-$C_{10}$, linear, or saturated or unsaturated, branched or cyclic $C_3$-$C_{20}$, preferably $C_3$-$C_{10}$, hydrofluoroalkyl or perfluoroalkyl radical; the hydrocarbon-based chain constituting said radicals optionally being interrupted with 1, 2, 3 or more heteroatoms chosen from:
  - oxygen,
  - sulfur,
  - nitrogen, and
  - silicon,

  and optionally being substituted with at least one radical chosen from:

  - $-OR_4$,
  - $-SR_4$,
  - $-NR_4R_5$,
  - $-COOR_4$,
  - $-CONHR_4$,
  - -CN,
  - a halogen atom,
  - a $C_1$-$C_6$ hydrofluoroalkyl or perfluoroalkyl radical, and/or
  - a $C_3$-$C_8$ cycloalkyl radical,

  with $R_4$ and $R_5$ representing, independently of each other, a hydrogen atom or a saturated $C_1$-$C_{30}$, in particular $C_1$-$C_{12}$, or unsaturated $C_2$-$C_{30}$, in particular $C_2$-$C_{12}$, linear, or a saturated or unsaturated, branched or cyclic $C_3$-$C_{30}$, in particular $C_3$-$C_{12}$, alkyl, perfluoroalkyl or hydrofluoroalkyl radical; or a $C_6$-$C_{10}$ aryl radical,
- X represents a radical chosen from:

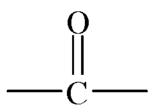

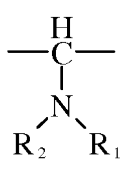

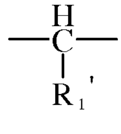

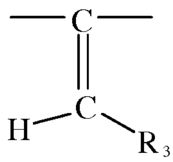

with $R_1$, $R_2$ and $R_3$ representing, independently of each other, a hydrogen atom or a radical R, with R as defined above, and $R'_1$ represents a hydrogen atom, an -OH group or a radical R as defined above, $R_1$ optionally also denoting a $C_6$-$C_{10}$ aryl radical;

- S represents a monosaccharide or a polysaccharide comprising up to 20 sugar units and in particular up to 6 sugar units, in pyranose and/or furanose form and of L and/or D series, said mono- or polysaccharide possibly being substituted with a mandatorily free hydroxyl group, and optionally one or more optionally protected amine function(s), and
- the bond S-$CH_2$-X represents a bond of C-anomeric nature, which may be α or β, and the cosmetically acceptable salts, solvates such as hydrates, and isomers thereof.

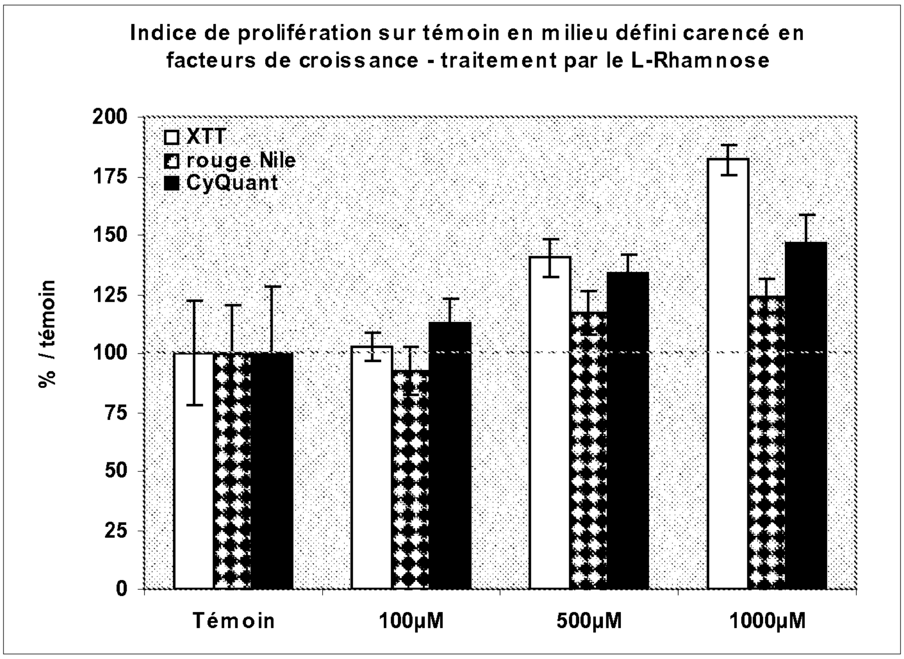


FIGURE 1

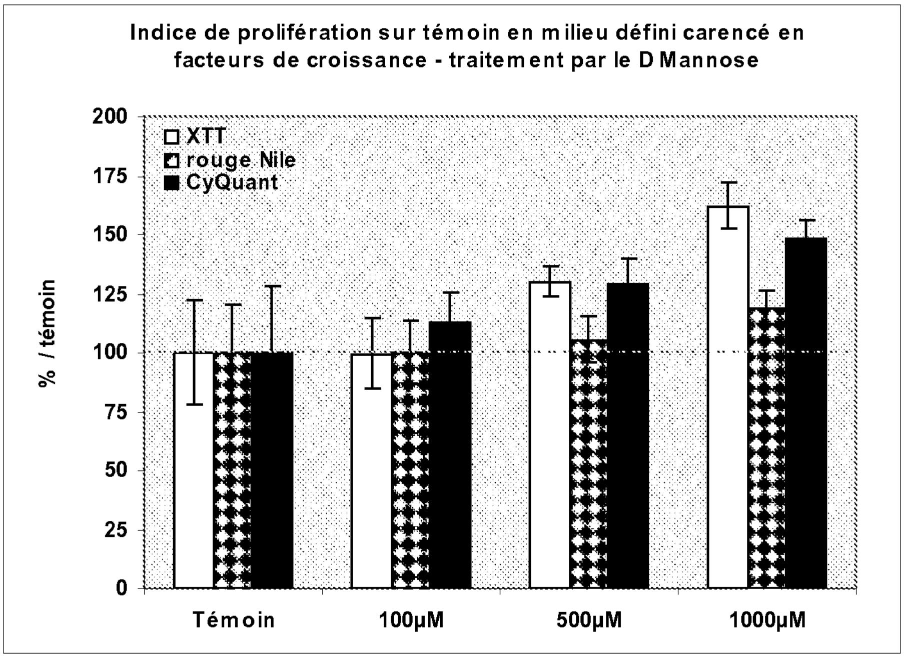


FIGURE 2

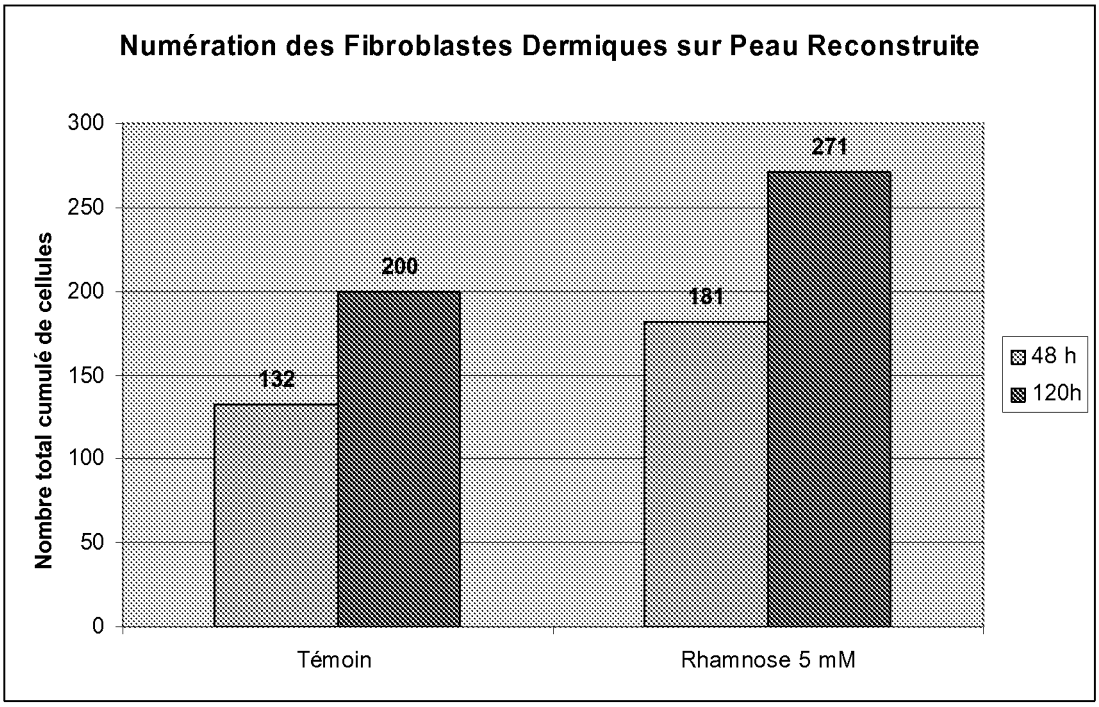


FIGURE 3

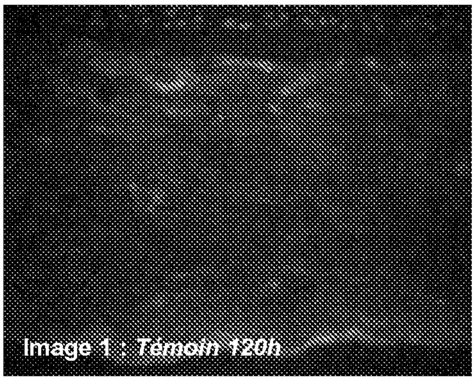


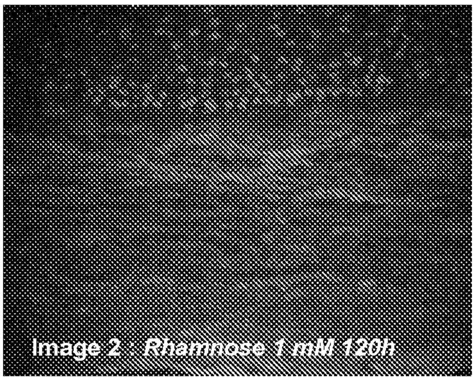


FIGURE 4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2007128939 A
- WO 2005063194 A
- WO 2002051828 A
- WO 02051828 A
- FR 2709666
- FR 2725369
- JP 2295912 A
- US 5412004 A
- US 5811487 A
- FR 2760641
- FR 2853543
- FR 2819175
- FR 2802425
- FR 2810548
- FR 2796278
- FR 2802420
- FR 2812544 A
- FR 2814950 A
- WO 0194381 A
- FR 2877220
- FR 2722380
- WO 0103538 A


### Littérature non-brevet citée dans la description


- **AUMAILLEY M ; ROUSSELLE P.** laminins of the dermoepidermal junction. *Matrix Biology,* 1999, vol. 18, 19-28
- **NIEVERS M ; SCHAAPVELD R ; SONNENBERG A.** Biology and function of hemidesmosomes. *Matrix Biology,* 1999, vol. 18, 5-17
- *Eur. J. Dermatol.,* 2008, vol. 18 (1), 36-40
- *Eur. J. Dermatol.,* 2008, vol. 18 (2), 297-302
- **BELL E. et al.** The reconstitution of living skin. *J Invest Dermatol,* Juillet 1983, 81
- **ASSELINEAU et al.** Models in Dermato. 1987, vol. III, 1-7